# EUROPEAN PATENT APPLICATION

(11) **EP 3 913 048 A1**
(43) Date of publication of application: **24.11.2021**
(21) Application number: 20382432.1
(22) Date of filing: 21.05.2020
(51) Int. Cl.: C12N 5/078, C12N 5/0783, C12N 5/00

(54) **A SYNTHETIC HYDROGEL AND ITS USE FOR IMMUNOTHERAPY AND 3D-PRINTING**

(71) Applicant: Consejo Superior de Investigaciones Científicas (CSIC), 28006 Madrid (ES); Consorcio Centro de Investigación Biomédica en Red, M.P. (CIBER), 28029 Madrid (ES); Fundació Institut de Bioenginyeria de Catalunya (IBEC), 08028 Barcelona (ES); Universitat politécnica de Catalunya (UPC), 08034 Barcelona (ES)
(72) Inventor: GUASCH CAMELL, Judit, 08193 Cerdanyola del Vallès (Barcelona) (ES); RATERA BASTARDAS, Imma, 08193 Cerdanyola del Vallès (Barcelona) (ES); VECIANA MIRÓ, Jaume, 08193 Cerdanyola del Vallès (Barcelona) (ES); PÉREZ DEL RÍO, Eduardo, 08193 Cerdanyola del Vallès (Barcelona) (ES); MARTINEZ MIGUEL, Marc, 08193 Cerdanyola del Vallès (Barcelona) (ES); RODRÍGUEZ RODRÍGUEZ, Xavier, 08193 Cerdanyola del Vallès (Barcelona) (ES); SANTOS ABREU, Roberto Fabião, 08193 Cerdanyola del Vallès (Barcelona) (ES); MATEOS TIMONEDA, Miguel Ángel, 28029 Madrid (ES); ENGEL LÓPEZ, Elisabeth, 08028 Barcelona (ES)
(74) Representative: Pons

(57) **Abstract**

The present invention discloses a hydrogel comprising a functionalized PEG multi-arm star polymer covalently combined with maleimide-functionalized low molecular weight heparin, further comprising at least one positively charged immune molecule, a method for producing this hydrogel and its use in T cell culture for immunotherapies. Furthermore, the invention relates to a bioink and its use in 3D-(bio)-printing.

## Description

The invention relates to a hydrogel formed with poly(ethylene)glycol (PEG) combined with heparin and a positively charged immune molecule, such as a cytokine or a cell-adhesive molecule, as well as its use in T cell culture and immunotherapies. Moreover, the invention relates to a (bio)ink that comprises said hydrogel and its use in 3D-(bio)printing.

### BACKGROUND ART

Immunotherapy is a medical strategy that offers a different approach to chemotherapy, radiation, and surgery in the treatment of diseases, particularly in cancer. Immunotherapy is based on employing and reinforcing the immune system of patients. The immune system protects the organisms against disease, distinguishing between self and non-self However, cancer appears from the organism own cells, and it creates a tumor microenvironment with immune evasion and immunosuppression signals capable of avoiding the immune response. The main objective of immunotherapy is to develop different strategies able to surpass cancer immunosuppression methods, detect and eliminate malignant cells without damaging healthy tissues.

Secondary lymphoid organs (SLOs) are in charge of generating and coordinating the immune responses in mammals. The lymph nodes (LNs) are SLOs that are positioned at strategic locations throughout the body connected through conduits filled with a fluid named lymph. The lymph contains a high concentration and transit of immune cells, especially T and B cells (lymphoid cells) that scan for the presence of their cognate antigen. SLOs development depends on the precisely regulated expression of cooperating lymphoid chemokines and cytokines. If T or B cells do recognize an antigen, the SLOs provide an optimal environment for cellular activation, proliferation, and selection for high affinity antibodies (Cupedo, T., et al 2012, Front. Immunol. 3, 343*).*

Cells and antigens also enter the LNs via an arteriole at the medullary sinus, which branches into a capillary bed with post-capillary venules, called high endothelial venules (HEVs). HEVs are placed in the T zone, and are the main points of entry for naïve T and B cells in response to 'homing' chemokines like CCL19 and CCL21.

Between all the immune cells, many immunotherapy techniques focus on modifying the activity of T cells to drive an anti-tumor response. The differentiation state and subset specification of T cells can influence their metabolism, cytotoxicity, and longevity.

Adoptive cellular therapy (ACT) is an approach that consists of using autologous T cells, which have been expanded in vitro, for a further reinfusion into the patient to treat cancer, autoimmune diseases, and even a few chronic illnesses. Initial ACTs in cancer only involved expanding ex vivo infiltrating tumor-specific T lymphocytes (TILs), relaying on the tumor specificity of these cells. However, recent advances have demonstrated that employing in vitro engineered T cells using engineered T cell receptors (TCR) or chimeric antigen receptors (CAR) may be an even better strategy to target cancer.

Most cell therapies, such as ACT, require a step of cell expansion. The improvement of this process could lead to the production of clinically relevant quantities of therapeutic cells, which is a current limitation that prevents their broad use in clinics. With the objective to overcome this challenge, mimicking the natural tissue of the immune cells and eventually engineering lymphoid tissues could help design better culture systems to provide higher proliferation rates and tune the resulting phenotypes. Moreover, a deeper understanding of the mechanisms that rule immune responses could be achieved.

The state of the art expansion systems in vitro use advanced biomaterials that replicate specific properties of the antigen-presenting cell (APC) surface. Such biomaterials are usually (micro)beads functionalized with ligands that specifically bind to known receptors on the T cell surface. The interaction between T cells and the artificial beads occurs in suspension media, totally overlooking the function of the SLOs.

Planar two dimensional (2D) surfaces have been shown to be valuable systems due to their capacity to mimic the in vivo environment in a well-controlled and simplified manner. For example a strategy employed surfaces coated with CCL21 and ICAM-1 together with IL-6 media enrichment as an ex vivo synthetic immune niche with the objective of effectively stimulating the proliferation of antigen-activated CD4+ T cells (Adutler-Lieber, S. et al. 2017, Blood Adv. 1, 1016-1030*).* Although a lot of valuable information was obtained with 2D studies, the natural T cell-APC interaction occurs in a three-dimensional (3D) space, the SLOs. Thus, 3D systems are need in order to efficiently replicate immune interactions in vitro.

A large variety of different 3D approaches have been studied to recreate the extracellular original conditions of many different cell lines in vitro, such as the formation of spheroids, the use of de-cellularized tissues, cell printing, and the culture of cells in 3D matrices using a high selection of different types of materials: natural and synthetic polymers, ceramics, metals, etc., all with their inherent advantages and disadvantages.

Synthetic polymers are attractive due to the possibility to control their chemical, structural, and physical properties, when the proper fabrication methods are used.

Nowadays, engineering a LN is more difficult than engineering larger organs like the heart or kidneys due to its high complexity resulting from their compartimentalization and cell density. Yet, the creation of an artificial ex vivo system for culturing immune cells that would mimic the LNs could be a powerful strategy to manipulate their behavior prior to adoptive immunotherapy. Different 3D scaffolds have been investigated to mimic primary and secondary lymphatic organs and/or their function.

For example, a sponge-like collagenous hydrogel was used to transplant a thymus-derived stromal cell line and dendritic cells (DCs) into mice renal subcapsular spaces, proving the generation of lymphoid tissue-like organoids with distinct compartmentalized B and T cell clusters and a similar CD4+/CD8+ ratio than the natural SLOs (Suematsu, S., et al, 2004, Nat. Biotechnol. 22, 1539-1545).

To mimic natural lymphocyte migration along collagen fibers, a synthetic collagen-mimetic peptide that binds to lymphocytes via the α2β1 collagen receptor, was integrated into polymerized alginate scaffolds used as implants for cell delivery *(*Stephan, S. B., et al. 2015, Nat. Biotechnol. 33, 97-101).

With the same objective, heparin-conjugated PEG hydrogels infused with collagen showed mechanical stability and the potential to depot supporting cytokines/chemokines. It was supported intra-scaffold migration of murine primary T cells and DCs, and when the cytokine CCL21 was bound to the structure, the motility of the T cells could be quantitatively compared to the in vivo migration observed in native SLOs (Stachowiak, A. N.,2008, J. Biomed. Mater. Res. A 85, 815-828).

However, the 3D scaffolds in the state of the art were not designed nor used to ex vivo produce the large amounts of specific T cells that are needed for immunotherapies in a short period and in an economically viable manner.

Accordingly, there is still the need of alternative 3D platforms or scaffolds for immunotherapy to those disclosed in the state of the art for culturing immune cells mimicking the extracellular matrix (ECM) of SLOs such as LNs, improving the proliferation, differentiation, replication and expansion of immune cells, particularly T cells. Moreover, there is still the necessity of designing and optimizing a biomaterial to turn the 3D scaffold into a dynamic material, which would allow its degradation as well as a material that can be used as a bioink for 3D bio-printing.

### SUMMARY OF THE INVENTION

The inventors functionalized heparin with maleimide and mixed it with solutions of different percentages of polyethyleneglycol (PEG) polymer to prepare a 3D PEG-Hep hydrogel. The hydrogel obtained with a 3%wt concentration of the PEG polymer was chosen due to its high pore size and connectivity. The inventors combined the 3%wt PEG hydrogel with different positively charged biomolecules such as cytokines and cell-adhesive molecules, in suspension or loaded to the hydrogels to provide alternative hydrogel scaffolds.

The highest proliferation parameters were achieved through the combination of a 3%wt PEG hydrogel with loaded CCL21 cytokine to the hydrogel and CCL19 cytokine added into the media. This scaffold proved to have potential to fabricate artificial LNs, mimic the ECM of LNs and overcome the limitations of current immunotherapies such as producing large amounts of T cells with therapeutic phenotypes. The inventors characterized and used the scaffold for T cell expansion, replication, and proliferation as well as phenotype tuning for applications in immunotherapy for cancer and autoimmune treatments.

Additionally, the inventors optimized the hydrogel gelification process to design a bioink for 3D printing. The inventors printed 3D scaffolds of a few layers, i.e. 4 and 6 layers, with the bioink and evaluated unloaded and loaded printed hydrogels for T cell culturing and ensuring their (bio)compatibility. The inventors observed higher rates of proliferation of T cells in scaffolds of 6 layers loaded with the CCL21 cytokine.

The present invention discloses a novel PEG-Hep hydrogel formed with a functionalized PEG polymer covalently combined with heparin, that further comprises at least one positively charged immune molecule, wherein it can be mimicked the ECM of LNs for T cell culture and used it in immunotherapy for cancer and autoimmune treatments. Thus the present invention discloses a bioink comprising said hydrogel, and its use in 3D bio-printing.

A first aspect of the present invention related to an hydrogel, hereinafter the hydrogel of the invention, comprising a functionalized PEG multi-arm star polymer covalently combined with heparin, forming a PEG-Hep hydrogel, further comprising at least one positively charged immune molecule.

As used herein, the term "hydrogel" refers to a 3D network of mixture of materials, molecules, polymers or substances that are combined by chemical bonds, including covalent, ionic and supramolecular bonds, or by any combination thereof, to form water-swellable but water-insoluble structures with a solid, semi-solid or semi-liquid texture.

The terms "poly(ethylene glycol)", "polyethyleneglycol" or "PEG" or "polyethylene oxide" or "polyoxyethylene" are considered equivalents and can be used interchangeably herein.

The term "multi-arm star" used herein refers to star-shaped polymers, which are the simplest class of branched polymers with a general structure consisting of at least three linear chains connected to a central core, wherein said core or the center of the polymer can be an atom, molecule, or macromolecule and the chains, or "arms", consist of variable-length organic chains. Star-shaped polymers in which the arms are all equivalent in length and structure are considered homogeneous, whereas the ones with variable lengths and structures are considered heterogeneous.

Preferably the PEG multi-arm star polymer, is functionalized with reactive end groups including, without limitation, N-hydroxysuccinimide ester, thiol, carboxyl, carbonyl, primary amine, aldehyde, anhydride, epoxide, maleimide, pyridyl disulfide, amines, hydrazides, isocyanate, sulfonyl chloride, fluorobenzene, imidoester, haloacetyl, vinylsulfone, carbodiimide, alkoxyamines, or iodoacetyl.

More preferably, the PEG-multi-arm polymer in the hydrogel of the invention is functionalized with thiol groups forming thiolated PEG or methoxy PEG thiol or methoxypolyethylene glycol thiol or mPEG thiol, wherein these terms are considered equivalents and can be used interchangeably herein.

Therefore, in a preferred embodiment the hydrogel of the invention comprises a thiol-functionalized PEG multi-arm star polymer, hereinafter PEG-SH.

In another preferred embodiment, the functionalized PEG multi-arm star polymer is a functionalized PEG four-arm star polymer; and more preferably is a thiol-functionalized PEG four-arm star polymer of formula:

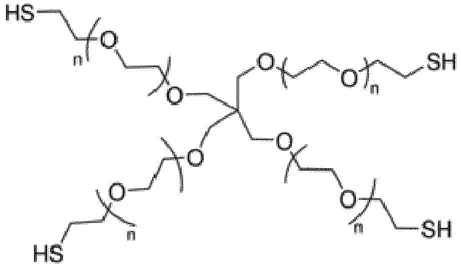

The concentration of the functionalized PEG multi-arm star polymer in the hydrogel of the invention comprising a certain range. In a preferred embodiment of the invention, the concentration of the functionalized PEG multi-arm star polymer in the hydrogel of the invention ranges between 2%wt to 10%wt on the total weight percentage of hydrogel.

The terms "% by weight" or "% wt" or "%w/w" are considered equivalents and can be used interchangeably herein and refer to the weight percentage relative to the total weight of the solution or dispersion, unless otherwise specified.

In another preferred embodiment of the hydrogel of the invention the functionalized PEG multi-arm star polymer concentration is selected from 2%wt, 2.5%wt, 3%wt, 3.5%wt, 4%wt, 4.5%wt, 5%wt, 5.5%wt, 6%wt, 6.5%wt, 7%wt, 7.5%wt, 8%wt, 8.5%wt, 9%wt, 9.5%wt and 10%wt.

The hydrogel of the invention comprising a 3%wt concentration of the functionalized PEG multi-arm star polymer presents higher pore size and connectivity, so in another more preferred embodiment of the hydrogel of the invention the functionalized PEG multi-arm star polymer concentration is 3%wt.

Further, the hydrogel of the invention comprises heparin.

The term "heparin" used herein, refers to a polysaccharide of a variably sulfated repeating disaccharide unit and highly negatively charged, which is a member of the glycosaminoglycan family of carbohydrates, wherein its molecular weight ranges from 3 to 30 kDa. A fractionated version of heparin, known as low molecular weight heparin (LMWH), comprising short chains and an average molecular weight of less than 8 kDa and for which at least 60% of all chains have a molecular weight lower than 8 kDa, preferably the average molecular weight is between 3 to 6 kDa.

It is routine practice for an expert in the art the methods of fractionation or depolymerization of heparin into low molecular weight heparin. Examples of methods of depolymerization include, without limitation, methanolysis, hydrolysis, glycolysis or aminolysis.

In another preferred embodiment the hydrogel of the invention comprises low molecular weight heparin (LMWH).

Preferably, the hydrogel of the invention comprises LMWH functionalized, and more preferably LMWH functionalized with the maleimide group forming the maleimide-functionalized low molecular weight heparin (Hep-Mal).

So, in another more preferred embodiment the hydrogel of the invention comprises maleimide-functionalized low molecular weight heparin, hereinafter Hep-Mal.

The functionalized PEG multi-arm star polymer is covalently combined with Hep-Mal forming a PEG-Hep hydrogel. The PEG-Hep hydrogel is formed through a reaction between the maleimide of the functionalized heparin and the reactive end groups of the functionalized PEG-multi-arm, that result in a covalent crosslink and the consequent gelation. The reactive end group of the functionalized PEG multi-arm have been previously described herein and apply equally to this embodiment.

In another preferred embodiment of the hydrogel of the invention, the reactive end group of the functionalized PEG multi-arm is a thiol, primary amine or an epoxide group, more preferably a thiol group. So, the PEG-Hep hydrogel is formed through a maleimide-thiol covalent reaction between the maleimide of the functionalized heparin and the thiols of the functionalized PEG multi-arm.

To prepare PEG-Hep hydrogels, a solution of functionalized PEG multi-arm star polymer is mixed with a solution of functionalized heparin in a proportion PEG-multi-arm:HEP-Mal which, for such calculation, five maleimide groups per molecule of heparin were estimated to react with the four functionalized groups of PEG-multi-arm, independently the concentration in weight (%wt) used of PEG-multi-arm.

In another preferred embodiment of the hydrogel of the invention the proportion PEG-multi-arm:HEP-Mal is 1:2, preferably 1:1.5.

The optimal pore size of the hydrogel of the invention allows the circulation through the inner part of the hydrogel immune cells as well as their proliferation, expansion, replication and differentiation showing the importance of the porosity of the hydrogel for mimicking the ECM of the LNs.

The terms "median pore size" or "median diameter size", used interchangeably herein, refer to the mean of the measure of the void or pore diameter, or the fraction of the volume of the voids over the total volume or the distance between two opposite walls of the void in the hydrogel of the invention.

It is routine practice for an expert in the art the methods of pore size measure or porosity measure. Examples of direct methods to measure porosity include, without limitation, optical and fluorescence methods, computed tomography methods, imbibition methods, water evaporation methods, mercury intrusion porosimetry methods, gas expansion methods, thermoporosimetry methods, physisorption methods, density methods, petrographic methods or electron microscopy such as (cryo) scanning electron microscopy or environmental electron microscopy. Examples of indirect methods to measure porosity include, without limitation, rheology measurements.

In another preferred embodiment of the hydrogel of the invention, it has a median pore size between 5 to 105 µm.

In another more preferred embodiment of the hydrogel of the invention, it has a median pore size between 15 to 95 µm, more preferred between 25 to 85 µm.

In another even more preferred embodiment of the hydrogel of the invention the median pore size is 30 µm, 35 µm, 40 µm, 45 µm, 50 µm, 55 µm, 60 µm, 65 µm, 70 µm, 75 µm, or 80 µm. In another even more preferred embodiment of the hydrogel of the invention the median pore size is 55 µm.

SLOs are in charge of generating and coordinating the immune responses in mammals, wherein there is a high concentration and transit of immune cells. In the SLOs, T and B cells scan for the presence of their cognate antigen. If T or B cells do recognize an antigen, the SLOs provide an optimal environment for cellular activation, proliferation, and selection for high affinity antibodies. These processes need to be reproduced in vitro for obtaining large amounts of therapeutic T cells in a short period of time and in an economic viable manner to ensure the success of cellular immunotherapies for cancer and autoimmune diseases.

The capacity of the heparin present in the hydrogel of the invention for anchoring positively charged biomolecules by electrostatically interactions, mimics the natural function of the heparin sulphates present in the ECM of the LNs.

For this reason, the hydrogel of the invention comprises at least one positively charged immune molecule.

The term "positively charged immune molecule" used herein, refers to molecules, particularly proteins, that are involved in mounting the immune response, especially the adaptive immune response associated with lymphocyte activation, and in particular T cell activation, which results in intracellular signalling events and inflammation, and have a positive net electrical charge for anchoring with the hydrogel of the invention. Examples of positively charged immune molecules include, without limitation chemokines, cytokines, immunoglobulins or antibodies, antigens and antigen presenting molecules, co-receptors, co-stimulatory molecules, checkpoint inhibitors, cell adhesion molecules, or growth factor receptors.

In another preferred embodiment of the hydrogel of the invention, the positively charged immune molecule is a cytokine and/or a cell adhesion molecule.

The term "cytokine" used herein, refers to small proteins or glycoproteins having a molecular mass of less than about 30 kDa, that are produced by cells of the immune system and that regulate or modulate inflammation, cancer or immune responses, such as modulation of the effector function of T cells, B cells, natural killers (NK) cells macrophages, APC or other immune system cells.

The hydrogel of the invention includes cytokines, which in turn include chemokines, interferons, interleukins and lymphokines.

The term "interferons" or "IFNs" used herein, refers to a group of signalling proteins made and released by host cells in response to the presence of several pathogens, such as viruses, bacteria, parasites, and tumor cells, and interfered in the viral replication and activated immune cells by up-regulating antigen presentation. Examples of interferons include, without limitation, the interferons belonging to the three classes: Type I IFN, Type II IFN, and Type III IFN.

The term "interleukins" or "ILs" used herein, refers to a group of cytokines that are expressed by white blood cells (leukocytes), synthesized by helper T cells, as well as by monocytes, macrophages, and endothelial cells. They promote the development and differentiation of T and B lymphocytes, and hematopoietic cells. Examples of interleukins include, without limitation, the families of interleukins: IL1, IL2, IL3, IL4, IL5, IL6, IL7, IL8, IL9, IL10, IL11, IL12, IL13, IL14, IL15, IL16 and IL17.

The term "lymphokines" used herein, refers to a group of cytokines that are produced by immune cells known as lymphocytes. They have many roles, including the attraction of other immune cells, including macrophages and other lymphocytes, to an infected site and their subsequent activation to prepare them to mount an immune response.

The term "chemokines" used herein, refers to a group of cytokines that are chemoattractant and guide the migration of cells, i.e. have the ability to induce directed chemotaxis and recruit cells of the immune system to a site of infection and promote the antigen-immune cell interaction. Chemokines have been classified into four main subfamilies: CXC, CC, CX3C and XC.

In another preferred embodiment of the hydrogel of the invention the cytokine is a chemokine.

In another more preferred embodiment of the hydrogel of the invention, the chemokine belongs to the CXC family and is selected from the list consisting of: CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCL17 and any combination thereof.

In another more preferred embodiment of the hydrogel of the invention, the chemokine belongs to the CX3C family and is CX3CL1.

In another more preferred embodiment of the hydrogel of the invention, the chemokine belongs to the XC family and is XCL1 and/or XCL2.

In another more preferred embodiment of the hydrogel of the invention, the chemokine belongs to the CC family and is selected from the list consisting of: CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL23, CCL24, CCL25, CCL26, CCL27, CCL28 and any combination thereof.

Chemokine ligand 21 (CCL21) and chemokine ligand 19 (CCL19) are small cytokines involved in the activation process of the immune system. These chemokines play an important role in costimulating the expansion of T cells and inducing T cell polarization and T cell motility. Both interact with the CCR7 receptor however, slight conformational changes in CCR7 following binding by the different chemokines result in differential T cell signalling.

Thus, in another even more preferred embodiment of the hydrogel of the invention the chemokine is CCL21 and/or CCL19.

In another even more preferred embodiment the hydrogel of the invention comprises the chemokines CCL21 and CCL19.

As expert in the art knows, a fragment of protein can be active, operative and functional such a native protein. So, in another more preferred embodiment of the hydrogel of the invention comprises at least one active fragment of the cytokines.

The terms "active fragments" used herein refers to a fragments, portion or peptides having substantially the same amino acid sequence of the polypeptide of the native cytokines described in previous embodiments of the invention, that are immunomodulatory active, operative and functional, i.e. can regulate or modulate the inflammation, cancer or immune responses as well as native cytokine. It is routine practice for an expert in the art to determinate the active fragments of the cytokines that are immunomodulatory active, operative and functional.

In another even more preferred embodiment of the hydrogel of the invention comprises at least one active fragment of chemokine.

The concentration of cytokine or the concentration of active fragments of cytokine in the hydrogel of the invention can be determined by a skilled in the art. In another preferred embodiment of the hydrogel of the invention, the concentration of cytokine is between 0.1 ng/mL to 250 ng/mL.

In another more preferred embodiment, the concentration of cytokine is between 50 ng/mL to 200 ng/mL.

In another even more preferred embodiment the concentration of cytokine is 50 ng/mL, 55 ng/mL, 60 ng/mL, 65 ng/mL, 70 ng/mL, 75 ng/mL, 80 ng/mL, 85 ng/mL, 90 ng/mL, 95 ng/mL, 100 ng/mL, 105 ng/mL, 110 ng/mL, 115 ng/mL, 120 ng/mL, 125 ng/mL, 130 ng/mL, 135 ng/mL, 140 ng/mL, 145 ng/mL, 150 ng/mL, 155 ng/mL, 160 ng/mL, 165 ng/mL, 170 ng/mL, 175 ng/mL , 180 ng/mL , 185 ng/mL , 190 ng/mL, 195 ng/mL or 200 ng/mL.

In another even more preferred embodiment, the concentration of cytokine is 100 ng/mL.

The positively charged immune molecule of the hydrogel of the invention can be a cell adhesion molecule. The term "cell adhesion molecule" used herein, refers to molecules, particularly proteins, located on the cell surface involved in binding with other cells or with the ECM in the cell adhesion process affecting cellular mechanisms including, without limitation, growth, migration, proliferation, contact inhibition, differentiation or apoptosis. Examples of cell adhesion molecules include, without limitation, integrin ligands such as ECM proteins like fibronectin, vitronectin, laminin, collagen, intercellular adhesion molecules (ICAMs), vascular cell adhesion molecule (VCAM), cadherins or selectins.

In another preferred embodiment of the hydrogel of the invention, the cell adhesion molecule is an intercellular adhesion molecule selected from the list consisting of: ICAM-1, ICAM-2, ICAM-3, ICAM-4, ICAM-5 and any combination thereof.

In another more preferred embodiment of the hydrogel of the invention, the intercellular adhesion molecule is ICAM-1.

As expert in the art knows, a fragment of protein can be active, operative and functional such a native protein. So, in another more preferred embodiment of the hydrogel of the invention comprises at least one active fragment of the cell adhesion molecule.

The terms "active fragments" has been defined in previous paragraphs of the present document, and apply equally to this aspect of the invention.

In another even more preferred embodiment, the hydrogel of the invention comprises at least one active fragment of a cell adhesion molecule, preferably of an intercellular adhesion molecule.

The concentration of cell adhesion molecule or the concentration of active fragments of cell adhesion molecule in the hydrogel of the invention can be determined by a skilled in the art. In another preferred embodiment of the hydrogel of the invention, the concentration of cell adhesion molecule ranges between 1 µg/mL and 50 µg/mL.

In another more preferred embodiment, the concentration of cell adhesion molecule ranges between 1 µg/mL and 25 µg/mL.

In another even more preferred embodiment, the concentration of cell adhesion molecule is 5 µg/mL, 10 µg/mL, 15 µg/mL or 20 µg/mL.

In another even more preferred embodiment the concentration of cell adhesion molecule is 5 µg/mL.

The positively charged immune molecule or the active fragments of the positively charged immune molecule can be linked or attached on a surface or material or in a solution or suspension. So that, in another preferred embodiment of the hydrogel of the invention the positively charged immune molecule is surface-immobilized or in a suspension. Also, in another preferred embodiment of the hydrogel of the invention the active fragment of the positively charged immune molecule is surface-immobilized or in a suspension.

The term "surface-immobilized" used herein refers to the attachment, fixation, linkage, adhesion, binding or coupling of the positively charged immune molecule to an inert surface, material, matrix or support, wherein the term "inert" refers to a not chemically reactive with the positively charged immune molecule. The material or support for positively charged immune molecule immobilization can be natural or organic, inorganic or a synthetic molecule or compound. Examples of materials for positively charged immune molecule immobilization include, without limitation, alginate, chitosan, chitin, collagen, gelatin, cellulose, starch, pectin, zeolites, ceramics, silica, glass, activated carbon, charcoal, polyvinyl chloride (PVC), oxides, titanium, aluminium, zirconium oxides, gold nanoparticles, titania nanoparticles or graphene.

It is routine practice for an expert in the art the methods of immobilization. Examples of the methods of immobilization include, without limitation, adsorption, covalent binding, coordination binding, entrapment, copolymerization, encapsulation or deposition.

The positively charged immune molecule of the hydrogel of the invention can also be in a cell suspension. The term "in suspension" used herein refers to a heterogeneous mixture or composition that contains solid or solute particles that do not dissolve, hereinafter the dispersed phase, and they are dispersed throughout the external phase, fluid or solvent, hereinafter the dispersion medium. Preferably, the dispersed phase of the suspension is a liquid, a semi-liquid, a semi-solid or a solid, and the dispersion medium is a liquid, a semi-liquid or a gas. More preferably the dispersed phase comprises immune cells and the dispersion medium comprises a positively charged immune molecule or an active fragment of a positively charged immune molecule, hereinafter the "positively charged immune molecule suspension".

It is routine practice for an expert in the art to determine the positively charged immune molecule suspension used in the hydrogel of the invention. Examples of suspension include, without limitation, emulsion, gel, sols, aerosol, foam, colloid or hydrosol.

According to the conventional techniques known to those skilled in the art, in case of a cell culture use, the hydrogel of the invention can further comprise supplementary compounds to increase cell viability, culture longevity and enhance the productivity without any significant adverse effect. Examples of supplementary compounds include, without limitation, magnesium salts, calcium salts, PHA, phorbol 12-myristate 13-acetate (PMA), ionomycin, brefeldin A, monensin, antibiotics, aminoacids, vitamins or hormones.

As a person skilled in the art knows, the hydrogel of the invention can be comprised in a composition. So, in another aspect, the present invention relates to a composition, hereinafter "composition of the invention", comprising the hydrogel of the invention.

According to the conventional techniques known to those skilled in the art, the composition according to the present invention may be formulated with an excipient and/or carrier. Thus, in a particular embodiment, the composition of the invention comprises an excipient and/or carrier. In case of a therapeutic use of the composition of the invention, this may be formulated with a pharmaceutically acceptable excipient and/or carrier.

The term "excipient" refers to a substance which helps to absorb any of the components of the composition of the invention, stabilizes said components or helps in the preparation of the composition in the sense of giving it consistency or, if necessary, providing flavors which make them more pleasant. Thus, excipients could have the function of keeping the components bound together, such as for example starches, sugars or celluloses, a sweetening function, a colorant function, the function of protecting the medicament, such as for example isolating it from the air and/or moisture, a filler function for a tablet, capsule or any other form of formulation, such as for example dibasic calcium phosphate, a disintegrating function to facilitate the dissolution of the components and their absorption in the intestine, without excluding other types of excipients not mentioned in this paragraph. Therefore, the term "excipient" is defined as any material included in the galenic forms which is added to the active ingredients or to its associations to enable its preparation and stability, modify its organoleptic properties or determine the physical/chemical properties of the composition and its bioavailability. The "pharmaceutically acceptable" excipient should allow for the activity of the compounds of the pharmaceutical composition, that is to say, for it to be compatible with said components. Examples of excipients are agglutinants, fillers, disintegrators, lubricants, coaters, sweeteners, flavorings and colorants. Nonlimiting, more specific examples of acceptable excipients are starches, sugars, xylitol, sorbitol, calcium phosphate, steroid fats, talc, silica or glycerin, amongst others.

The term "carrier" refers to a compound which facilitates the incorporation of other compounds to allow a better dosing and administration or to give consistency and form to the composition. Therefore, the carrier is a substance which is used to dilute any of the components of the composition of the present invention to a determined volume or weight, or even without diluting said components, capable of allowing better dosing and administration or giving consistency. When the form of presentation is liquid, the carrier is the diluent. In a particular embodiment, the carrier is pharmaceutically acceptable.

As explained previously at the present description, the inventors have developed an alternative scaffold that comprises a PEG-Hep hydrogel and positively charged immune molecules, mimicking the ECM of LNs for T cell expansion, replication and proliferation, fabrication of artificial LNs and producing large amounts of T cells with therapeutic phenotypes.

Consequently, another aspect of the invention is the hydrogel of the invention or the composition of the invention for use as a medicament.

In another aspect of the invention, the hydrogel of the invention or the composition of the invention for use in immunotherapy, preferably in cancer treatments and autoimmune diseases.

The term "treating" (or "treat" or "treatment") used herein refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders associated with immune diseases or cancer.

The terms "immunotherapy", "biotherapy", "biological therapy", "biological response modifying therapy" or "MRB therapy" used herein, refer to the set of treatment strategies to stimulate, inhibit or replenish the immune system against cancer, infections or other diseases as well as to lessen the side effects of very aggressive treatments used against cancer.

Another preferred embodiment is the hydrogel of the invention or the composition of the invention for use in T cell based adoptive immunotherapy.

The terms "T cell based adoptive immunotherapy", "adoptive cell transfer", "adoptive cell therapy" or "ACT" are considered equivalents and can be used interchangeably herein, refer to a therapeutic approach which involves the isolation and reinfusion or transfer of T lymphocyte immune cells into patients to treat disease, preferably to treat cancer or autoimmune disease. Types of adoptive cell therapy include chimeric antigen receptor T cell (CAR T cell) therapy, T cell receptor (TCR) gene-modified T cell therapy, tumor-infiltrating lymphocyte (TIL) therapy, lymphokine-activated killer (LAK) cell therapy and cytokine-induced killer (CIK) cell therapy. The cells may have originated from the patient or from another individual. In autologous cancer immunotherapy, cells are extracted from the patient, cultured in vitro and returned to the same patient. Comparatively, allogeneic therapies involve cells isolated and expanded from a donor separate from the patient receiving the cells.

Examples of types of cancer suitable for immunotherapy include, without limitation, melanoma, colorectal carcinoma, cervical cancer, lymphoma, leukaemia, bile duct cancer, neuroblastoma, lung cancer, breast cancer or sarcoma.

Cellular immunotherapies require cell culture, and activation, expansion, proliferation and differentiation of the immune cells, preferably of the T cells. The higher immune cell proliferation rates is relevant for producing necessary quantities of therapeutic cells to reach the adequate clinical doses. As described previously, by the hydrogel of the invention, the inventors obtained high proliferation parameters and produced large amounts of T cells.

Thus, another aspect of the invention is the use of the hydrogel of the invention or the composition of the invention for cell culture, preferably for immune cell culture and more preferably for T cell culture.

In another more preferred embodiment is the use of the hydrogel of the invention or the composition of the invention for activation, expansion, proliferation and/or differentiation of T cells.

The term "T cell activation" used herein refers to a process which T cells identify small numbers of specific foreign antigens, usually peptides, through receptors at the surface and lead to proliferation and differentiation of T cells.

The terms "T cell expansion" or "T cell replication" used herein refer to a process or response of expanding, widening or extending the T cell populations which correlates with the proliferative process. The expansion and replication parameters correlate with a high quantity of cells after the proliferative process.

The term "T cell proliferation" used herein refers to the cell division and cell growth of the cells from the original population to increase the number of T cells a new have undergone divided by the number of divided cells.

The term "T cell differentiation " used herein refers to the process in which a T cell changes from one T cell phenotype to another more specialized or mature. Examples of T cell differentiation states include, without limitation, naïve T cells, central memory T cells, memory T cells or effector T cell.

The T cells originate as precursor cells, derived from bone marrow, and develop into several distinct types of T cells once they have migrated to the thymus gland. T cell differentiation continues even after they have left the thymus. T cells are grouped into a subsets based on their function.

Types of T cells include, without limitation, helper CD4+ T cells, cytotoxic CD8+ T cells, regulatory T cells, natural killer T cells (NK T cells), memory T cells and gamma delta T cells (γδ T cells).

The "T helper cells", "Th cells" or "CD4+ T cells", terms used interchangeably herein, refer to a type of T cell that expresses the surface protein CD4 and play an important role in the immune system, particularly in the adaptive immune system. They help the activity of other immune cells to suppress or regulate immune responses to pathogens and tumor cells. This anti-tumor activity of the CD4+ T cell is used in many immunotherapy techniques, like adoptive cellular therapy (ACT), where autologous cancer-reactive T cells are reinfused into the patient after an ex vivo treatment for tumor destruction.

Consequently, another more preferred embodiment is the use of the hydrogel of the invention or the composition of the invention for CD4+ T cell culture.

In another even more preferred embodiment is the use of the hydrogel of the invention or the composition of the invention for activation, expansion, proliferation and/or differentiation of CD4+ T cells.

The inventors optimized the hydrogel of the invention to design a bioink for 3D printing and evaluated the printed hydrogels to observe their effect on immune cell culturing and ensure their (bio)compatibility.

Consequently, another aspect of the present invention relates to a bioink, which comprises the hydrogel of the invention or the composition of the invention, hereinafter the bioink of the invention.

The term "bioink" used herein refers to a composition, substance, material or compound that can be used for 3D printing of complex models or scaffolds that mimic an extracellular matrix environment to support the adhesion, proliferation, and differentiation of living cells, preferably of immune cells, more preferably of T cells.

Thus, another aspect of the present invention relates to the use of the hydrogel of the invention, composition of the invention or the bioink of the invention in 3D printing, preferably in 3D bio-printing.

The term "3D printing" used herein refers to a process to build a three-dimensional object from a computer-aided design (CAD) model, usually by successively adding material layer by layer, in which material is joined or solidified under computer control to create the 3D object or scaffold.

The term "3D bio-printing" used herein refers to the utilization of 3D printing like techniques to combine cells, growth factors, and biomaterials to fabricate a 3D object, part, tissue or structure that maximally imitate natural and biological characteristics. Preferably, 3D bio-printing utilizes the layer-by-layer method to deposit materials or bioinks to create structures that are later used in biotechnology, medicine and tissue engineering fields.

The inventors obtained the hydrogel of the invention by the combination of a functionalized PEG multi-arm star polymer with maleimide-functionalized low molecular weight heparin (Hep-Mal) and at least one positively charged immune molecule.

Thus, another aspect of the present invention is a method, hereinafter the method of the invention, for producing the hydrogel of the invention that comprises the following steps:
(a) mixing a solution of functionalized PEG multi-arm star polymer with another solution of heparin, both in buffer solution,
(b) incubating the solution obtained in step (a) at between 15°C to 45°C to gelify to form a hydrogel, and
(c) loading the hydrogel obtained in step (b) with at least one positively charged immune molecule.

The terms "hydrogel", "multi-arm star", "functionalized PEG polymer", "heparin" and "positively charged immune molecule" have been defined in previous paragraphs of the present document, and apply equally to this aspect of the invention, as well as to particular embodiments (alone or in combination).

It is routine practice for an expert in the art to determinate the buffer solutions compatible with functionalized PEG multi-arm star polymer and heparin. Examples of buffer solutions, without limitation, are phosphate buffered saline (PBS), Dubelcco's PBS, RPMI (Roswell Park Memorial Institute Medium) or DMEM (Dulbecco's Modification of Eagle's Medium) and combinations of these buffer with supplements such as salts, aminoacids and chemicals compounds.

Preferably the PEG multi-arm star polymer, is functionalized with reactive end groups including, without limitation, N-hydroxysuccinimide ester, thiol, carboxyl, carbonyl, primary amine, aldehyde, anhydride, epoxide, maleimide, pyridyl disulfide, amines, hydrazides, isocyanate, sulfonyl chloride, fluorobenzene, imidoester, haloacetyl, vinylsulfone, carbodiimide, alkoxyamines, or iodoacetyl.

More preferably, in another preferred embodiment of the method of the invention, the PEG-multi-arm polymer is functionalized with thiol groups forming a thiol-PEG multi-arm star polymer.

In another more preferred embodiment of the method of the invention, the functionalized PEG multi-arm star polymer is a functionalized PEG four-arm star polymer; and even more preferably is a thiol-functionalized PEG four-arm star polymer.

As in the hydrogel of the invention, the concentration of the functionalized PEG multi-arm star polymer in the solution, in the step (a) of the method of the invention, comprising a certain range. In another preferred embodiment of the method of the invention the concentration of the functionalized PEG multi-arm star polymer ranges between 2%wt to 10%wt on the total weight percentage of hydrogel.

The term "%wt" has been defined in previous paragraphs of the present document, and apply equally to this aspect of the invention.

In another preferred embodiment of the method of the invention the functionalized PEG multi-arm star polymer concentration is selected from 2%wt, 2.5%wt, 3%wt, 3.5%wt, 4%wt, 4.5%wt, 5%wt, 5.5%wt, 6%wt, 6.5%wt, 7%wt, 7.5%wt, 8%wt, 8.5%wt, 9%wt, 9.5%wt and 10%wt; the more preferred is 3%wt.

In step (a) of the method of the invention, the functionalized PEG multi-arm star polymer is covalently combined with heparin forming a PEG-Hep hydrogel, that results in a covalent crosslink and the consequent gelation.

In another preferred embodiment of the method of the invention the heparin is low molecular weight heparin (LMWH).

Preferably, the heparin of the method of the invention is functionalized low molecular weight heparin, and more preferably is maleimide-low molecular weight heparin (Hep-Mal).

A solution of functionalized PEG multi-arm star polymer is mixed with a solution of functionalized heparin in a proportion PEG-multi-arm:HEP-Mal 1:2. So, in another preferred embodiment of the method of the invention the proportion PEG-multi-arm:HEP-Mal in step (a) is 1:2, preferably 1:1.5.

In step (b) of the method of the invention, the hydrogel is formed. Further, in step (c) of the method of the invention, the hydrogel obtained in step (b) is loaded with at least one positively charged immune molecule.

The term "positively charged immune molecule" has been defined in previous paragraphs of the present document, and apply equally to this aspect of the invention.

In a preferred embodiment of the method of the invention, the positively charged immune molecule is a cytokine and/or a cell adhesion molecule.

The terms "cytokine" and "cell adhesion molecule" have been defined in previous paragraphs of the present document, and apply equally to this aspect of the invention.

The cytokines of the method of the invention include chemokines, interferons, interleukins and lymphokines, preferably chemokines.

The terms "chemokines", "interferons", "interleukins" and "lymphokines" have been defined in previous paragraphs of the present document, and apply equally to this aspect of the invention, as well as to particular embodiments (alone or in combination).

In another more preferred embodiment of the method of the invention, the chemokine belongs to the CC family and is selected from the list consisting of: CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL23, CCL24, CCL25, CCL26, CCL27, CCL28 and any combination thereof.

Thus, in another even more preferred embodiment of the method of the invention the chemokine is CCL21 and/or CCL19.

In another even more preferred embodiment of the method of the invention the chemokines are CCL21 and CCL19.

In another even more preferred embodiment of the method of the invention the hydrogel obtained in step (b) is loaded with at least one active fragment of the cytokines, more preferably at least one active fragment of the chemokines.

The term "active fragments" has been defined in previous paragraphs of the present document, and apply equally to this aspect of the invention, as well as to particular embodiments (alone or in combination).

The concentration of cytokine or the concentration of active fragments of cytokine in step (c) of the method of the invention can be determined by a skilled in the art. In another preferred embodiment of the method of the invention, the concentration of cytokine ranges between 0.1 ng/mL to 250 ng/mL.

In another more preferred embodiment, the concentration of cytokine ranges between 50 ng/mL to 200 ng/mL.

In another even more preferred embodiment the concentration of cytokine is 50 ng/mL, 55 ng/mL, 60 ng/mL, 65 ng/mL, 70 ng/mL, 75 ng/mL, 80 ng/L, 85 ng/mL, 90 ng/mL, 95 ng/mL, 100 ng/mL, 105 ng/mL, 110 ng/mL, 115 ng/mL, 120 ng/mL, 125 ng/mL, 130 ng/mL, 135 ng/mL, 140 ng/mL, 145 ng/mL, 150 ng/mL, 155 ng/mL, 160 ng/mL, 165 ng/mL, 170 ng/mL, 175 ng/mL , 180 ng/mL , 185 ng/mL , 190 ng/mL, 195 ng/mL or 200 ng/mL.

In another even more preferred embodiment the concentration of cytokine is 100 ng/mL.

In step (c) of the method of the invention, the hydrogel obtained in step (b) can be loaded with at least one cell adhesion molecule.

In another preferred embodiment of the method of the invention, the cell adhesion molecule is an intercellular adhesion molecule selected from the list consisting of: ICAM-1, ICAM-2, ICAM-3, ICAM-4, ICAM-5 and any combination thereof.

In another more preferred embodiment of the method of the invention, the intercellular adhesion molecule is ICAM-1.

As an expert in the art knows, a fragments of cell adhesion molecule can be active, operative and functional such a native protein. So, in another more preferred embodiment the method of the invention comprises at least one active fragment of a cell adhesion molecule, preferably at least one active fragment of an intercellular adhesion molecule.

The concentration of cell adhesion molecule or the concentration of active fragments of cell adhesion molecule in the method of the invention can be determined by a skilled in the art. In another preferred embodiment of the method of the invention, the concentration of cell adhesion molecule ranges between 1 µg/mL and 50 µg/mL, more preferred ranges between 1 µg/mL and 25 µg/mL.

In another even more preferred embodiment, the concentration of cell adhesion molecule is 5 µg/mL, 10 µg/mL, 15 µg/mL or 20 µg/mL.

In another even more preferred embodiment the concentration of cell adhesion molecule is 5 µg/mL.

The positively charged immune molecule or the active fragments of a positively charged immune molecule can be linked or attached on a surface or material or in a solution or suspension. Another preferred embodiment of the method of the invention wherein the positively charged immune molecule is surface-immobilized or in suspension.

The terms "surface-immobilized" and "suspension" have been defined in previous paragraphs of the present document, and apply equally to this aspect of the invention, as well as to particular embodiments (alone or in combination).

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and are provided by way of illustration and are not intended to be limiting of the present invention.

### DESCRIPTION OF THE DRAWINGS

**Figure 1****.** Spectrum of the low molecular weight heparin functionalized with maleimide (peak circled) after an overnight reaction.
**Figure 2****.** Scheme of a PEG-Hep hydrogel formation through a maleimide-thiol reaction resulting in a covalent crosslink and gelation.
**Figure 3****.** A) Strain sweeps and B) frequency sweeps of 6%wt, 4%wt and 3%wt of PEG-Hep hydrogels.
**Figure 4****.** Time sweeps of 6%wt, 4%wt and 3%wt PEG-Hep hydrogels.
**Figure 5****.** A) SEM images of the surface and section of the samples studied at different compositions of PEG (6%wt, 4%wt and 3%wt). B) Pore size evaluation of 6%wt, 4%wt and 3%wt PEG-Hep hydrogels. The statistical significance was determined by the non-parametric Kruskal Wallis ANOVA test (*** p < 0.001).
**Figure 6****.** A) Lateral and B) top views of an overall 3% PEG-Hep hydrogel of 1 cm of diameter obtained by X-ray microtomography. C) and D) show the same perspectives but of a small zone of the hydrogel used to analyzed its porosity of 3.5 mm of diameter and 500 µm of height.
**Figure 7****.** Porosity analysis of 3%wt PEG-Hep samples studied by microtomography (N(samples) = 2) and the Gaussian fitting performed.
**Figure 8****.** GFP loading curve of 6%wt, 4%wt and 3%wt of PEG-Hep hydrogels. The statistical significance was determined by the Mann-Whitney U test (*** p < 0.001).
**Figure 9****.** A) Normalized proliferation analysis of CD4+ T cells 5 days after seeding in unloaded PEG-Hep hydrogels (Ndonors = 6). Statistical significance was determined by the Mann-Whitney U test (*p<0.05, **p<0.01). B) Diagram of the resulting CFSE fluorescence peaks of a representative data point.
**Figure 10****.** Normalized proliferation analysis of CD4+ T cells 6 days after seeding in suspension (positive control) and with different concentrations of CCL21 (Ndonors = 5, with a minimum of Ndonors/condition = 4).

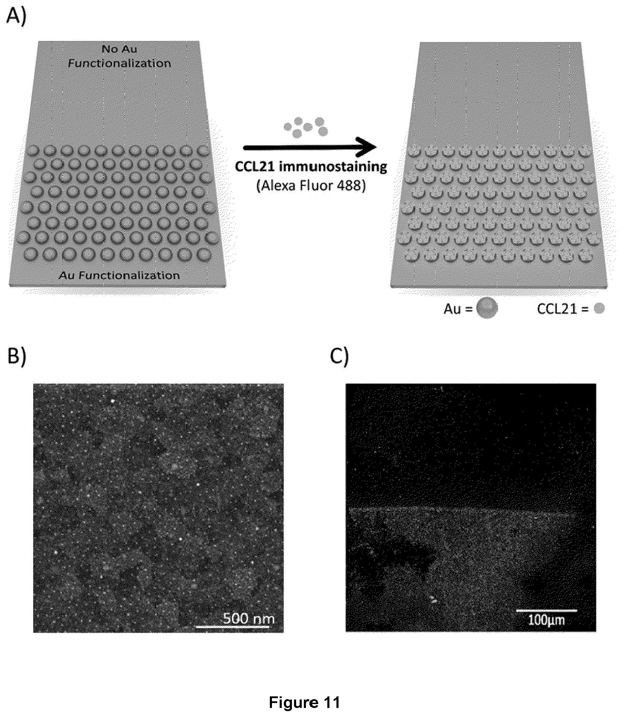

**Figure 11****.** A) Scheme of the experiment performed to study the adhesion of CCL21 onto gold substrates using an indium titanium oxide (ITO) substrate decorated with a quasi hexagonal array of gold nanoparticles (AuNPs) functionalized with CCL21 and further immunostained (Alexa Fluor 488). B) SEM image of the ITO substrate with the quasi-hexagonal array of (AuNPs). C) Fluorescence image of the surface area decorated with AuNPs, functionalized with CCL21, immunostained with human anti-CCL21 and the secondary antibody with mouse anti-human Alexa 488, showing signal only on its lower half, where de AuNPs are present.
**Figure 12****.** Normalized proliferation analysis of CD4+ T cells 6 days after seeding in suspension (positive control) and with different concentrations of CCL21 fixed to Au surfaces (Ndonors = 8). Statistical significance was determined by the Mann-Whitney U test (** p < 0.01).
**Figure 13****.** Normalized replication, expansion, and proliferation indexes 6 days after seeding CD4+ T cells in PEG-Hep hydrogels loaded with 100 ng/mL of CCL21 and unloaded (Ndonors = 18, with a minimum of Ndonors/condition = 6). Statistical significance was determined by the Mann-Whitney U test (*p<0.05, **p<0.01, ***p<0.001).
**Figure 14****.** A) Normalized proliferation indexes 6 days after seeding CD4+ T cells in PEG-Hep hydrogels loaded with 100 ng/mL of CCL21 (Ndonors = 6). Statistical significance was determined by the Mann-Whitney U test (*p<0.05, **p<0.01). B) Diagram of the resulting CFSE fluorescence peaks of a representative data point.
**Figure 15****.** Differentiation analysis of CD4+ T cells 5 days after seeding (Ndonors = 10, with a minimum of Ndonors/condition = 4). Percentage of A) naive (TN), B) central memory (TCM), and C) effector memory (TEM). Representative dot plot graphs of cells in a D) negative control, E) positive control, F) 3%wt PEG-Hep hydrogel, and G) 3%wt PEG-Hep hydrogel loaded with a solution of 100 ng/mL of CCL21. Statistical significance was determined by the Mann-Whitney U test (*p<0.05).
**Figure 16****.** Normalized proliferation analysis of CD4+ T cells 6 days after seeding with different concentrations of CCL19 in suspension (Ndonors =8). Statistical significance was determined by the Mann-Whitney U test (**p<0.01, ***p<0.001).
**Figure 17****.** Normalized proliferation indexes 6 days after seeding CD4+ T cells in PEG-Hep hydrogels loaded with 100 ng/mL and 20 ng/mL of CCL19 (Ndonors = 6). Statistical significance was determined by the Mann-Whitney U test (*p<0.05, **p<0.01).
**Figure 18****.** A) Normalized proliferation indexes 6 days after seeding CD4+ T cells in PEG-Hep hydrogels loaded with 100 ng/mL of CCL21 and with 100 ng/mL of CCL19 in solution (Ndonors = 6). Statistical significance was determined by the Mann-Whitney U test (*p<0.05, **p<0.01). B) Diagram of the resulting CFSE fluorescence peaks of a representative data point.
**Figure 19****.** Schematic images of the scaffold designed to 3D print structures.
**Figure 20****.** Microscope images of the resulting scaffolds printed with a 3%wt PEG-Hep hydrogel after A) 3.5 hours of gelation, B) one day at room temperature, and C) one day incubated at 37°C. D) Microscope images of scaffolds printed with 3%wt PEG-Hep hydrogels made in DMEM media instead of PBS.
**Figure 21****.** Normalized proliferation analysis of CD4+ T cells seeded in unloaded printed PEG-Hep hydrogels of 4 and 6 layers of height, 6 days after seeding (Ndonors = 6). Statistical significance was determined by the Mann-Whitney U test (*p<0.05, **p<0.01).
**Figure 22****.** Differentiation analysis of CD4+ T cells (Ndonors = 6). Percentage of A) naive (TN), B) central memory (TCM), and C) effector memory (TEM) for 3D structures consisting of 4 layers. Percentage of D) naïve (TN), E) central memory (TCM), and F) effector memory (TEM) for 3D structures consisting of 6 layers.
**Figure 23****.** A) Normalized proliferation indexes 6 days after seeding CD4+ T cells in PEG-Hep hydrogels loaded with 100 ng/mL of CCL21 (Ndonors = 6). Statistical significance was determined by the Mann-Whitney U test (*p<0.05, **p<0.01). B) Diagram of the resulting CFSE fluorescence peaks of a representative data point.
**Figure 24****.** Normalized proliferation analysis of CD4+ T cells seeded in unloaded hydrogels and loaded PEG-Hep hydrogels with 1, 5, and 50 µg/ml 6 days after seeding (Ndonors ≥ 2). Statistical significance was determined by the Mann-Whitney U test (**p<0.01).

### EXAMPLES

### MATERIALS AND METHODS

### 1. CHEMICAL TECHNIQUES: SYNTHESIS OF A PEG-HEP HYDROGEL

### 1.1. Materials

Green fluorescence protein (GFP) was synthesized as described in Unzueta, U. et al, Int. J. Nanomed. 7, 4533-4544 (2012). Low molecular weight heparin was purchased from Fisher Scientific (Fisher BioReagents, Spain). Boc-L-phenylalanine from Merk, Germany, and poly(ethylene oxide), 4-arm, thiol terminated (Mn=10000 g/mol), N-(2-aminoethyl) maleimide trifluoroacetate salt (AEM), 1-hydroxybenzotriazole hydrate (HOBT), N-(3-dimethylamino-propyl)-N'-ethylcarbodiimide hydrochloride (EDC·HCl), 2-(N-morpholino) ethanesulfonic acid (MES), and the rest of the products not otherwise specified were purchased from Sigma-Aldrich (USA).

### 1.2. Synthesis of heparin functionalized with maleimide

The functionalization of heparin with maleimide was based in a method described in Nie, T., et al 2009, Acta Biomater. 5, 865-875. Briefly, the inventors added 0.02 mmol of heparin in a solution of MES with HOBT (0.15 mmol), AEM (0.08 mmol), and EDC·HCl (0.11 mmol) and left it overnight. The product was purified by dialysis (MWCO 1000) against water, lyophilized, and characterized by proton nuclear magnetic resonance (1H-NMR) spectroscopy.

### 1.3. PEG-Hep hydrogel formation

To prepare PEG-Hep hydrogels, the inventors mixed a solution of 4-arm thiolated PEG (PEG-SH) with a solution of maleimide-functionalized low molecular weight heparin (Hep-Mal) in a proportion of 1:1.5, both in PBS. The inventors used different concentrations in weight of PEG: 6%wt, 4%wt, and 3%wt, to obtain different types of hydrogels. Once the solutions were mixed, they were kept in the incubator at 37 °C during at least 1 h to form the hydrogel. Negative controls consisting of solutions of only one of the reactants at relevant concentrations confirmed that gelification and therefore hydrogel formation were caused by the reaction between the thiol groups of the 4-arm PEG and the maleimide-functionalized heparin.

### 2. PHYSICAL TECHNIQUES

### 2.1. Rheology

The inventors used the small-amplitude oscillatory shear (SAOS) technique to characterize the linear-viscoelastic regime (LVE) of the hydrogels using a Rheometer HAAKE RheoStress RS600 (Thermo Fisher Scientific, USA). The experiments performed were strain sweeps, frequency sweeps, and time sweeps at 37°C to calibrate the range of pressure and frequency where the hydrogels maintain their viscoelastic behavior, characterize the gelification process, and achieve the value of the gel equilibrium shear modulus (Ge).

### 2.2. Scanning electron microscopy (SEM)

Although this technique is typically used in dry samples, a special protocol at the vacuum chamber was used, slowly decreasing the pressure and temperature, which enabled to image the structure of the hydrated hydrogels. The equipment used by the inventors was a FEI Quanta 650F Environmental scanning electron microscope.

### 2.3. Microtomography

Microtomography is an X-ray 3D imaging technique with a high resolution that allows the visualization of the internal structure of a sample. In this case, the inventors used a skyscan 1272 high-resolution micro computed tomography (Bruker).

### 2.4. Preparation of surfaces with AuNPs for CCL21 immobilization.

The inventors prepared nanostructures by block copolymer micellar lithography (BCML) as described in Guasch, J., et al 2016, Chem. Mater. 28, 1806-1815. BCML consists of dissolving an amphiphilic block copolymer in an apolar solvent to create reverse micelles, which can be loaded with a metallic precursor. The inventors dissolved polystyrene (x)-2-vinylpyridine (y) (PS(106)-P2VP(75) (Polymer Source Inc., Canada) in a dry solution of o-toluene at room temperature and stirred for 24h. Then, they added gold (III) chloride trihydrate (Sigma-Aldrich, USA) to the block copolymer micellar solution and stirred for 48 h. The inorganic metal complex compound is dissolved into the polar micellar cores, where it loses a proton that binds to the nitrogen atom of the P2VP chain, stabilizing the micelles.

The inventors dipped-coated commercial indium titanium oxide (ITO)-coated glass substrates (20 mm x 15 mm Ossila Ltd, UK) with the loaded gold micellar solution at a constant velocity of 110 mm/min and then plasma treated the sample with oxygen plasma (150 W, 0.15 mbar, 45 min) using a microwave gas plasma system (210 PVA TePla, Germany) to obtain quasi-hexagonally ordered AuNPs with lateral interparticle distances of 68 ± 20 nm. After that, the inventors passivated the ITO surface with PEG-silane (Prochimia, Poland), milliQ water, and triethylamine (Sigma Aldrich, USA) in toluene overnight at 80°C. Then, they functionalized it with CCL21 (Sigma Aldrich, USA) during 1 h at room temperature by means of the cysteine groups of the cytokine.

### 2.5. Preparation of Au surfaces. Gold evaporation

The inventors studied the function of CCL21 and its effect in T cell proliferation in suspension and immobilized on planar Au surfaces to analyze the influence of fixing this cytokine on CD4+ T cell proliferation. The inventors incubated it with CCL21 during 1 h at the desired concentration.

### 2.6. 3D Printing with PEG-Hep hydrogel

For these experiments, the inventors used 3%wt PEG-Hep hydrogels as ink. To make the 3D scaffolds, the inventors mixed sterilized solutions of PEG-SH and Hep-Mal a day before the impression in a sterile syringe adequate for the printing, and incubated the mixture at room temperature. After that, the inventors placed the syringe in the printer with a tip TIP27GA TT 008" NAT, which showed to be adequate for the printing of this material at a pressure of 1.2 bar, and an impression speed of 15 mm/s in a 24WP.

### 2.7. Loading capacity

The inventors loaded 3%wt PEG-Hep hydrogels with solutions at different concentrations of GFP during 1 h. After that, the inventors removed the supernatant and washed the hydrogels with PBS. The inventors measured the fluorescence of the hydrogels with a Victor 3 Multioption plate reader (Perkin Elmer, USA).

### 3. BIOLOGICAL TECHNIQUES

### 3.1. CD4+ T cell isolation and purification

The inventors obtained the primary human CD4+ T cells through a purification process of buffy coats of healthy adult donors, obtained from "Banc de Sang i Teixits" (Barcelona, Spain) after the approval of the "Ethics Committee on Animal and Human Experimentation" of the Autonomous University of Barcelona (No. 3511). The buffy coat is the fraction of an anticoagulated blood sample that contains the white blood cells and platelets. The inventors worked under a flow hood and used of sterile tools and materials.

To obtain the CD4+ T cells, they first purified the PBMCs by density gradient centrifugation using Ficoll. Briefly, the inventors diluted blood from the buffy coat with pre-warmed PBS with 2 mM of EDTA in a proportion of 1:4. Then, they added the Ficoll and centrifuged the mixture during 20 min at 300 g. Once the sample was centrifuged, the "white" phase between the supernatant (plasma) and the Ficoll was collected and washed with PBS with 2mM of EDTA. Afterwards, the inventors counted the achieved cells and used a CD4+ T cell isolation kit purchased from Miltenyi Biotec S. L. (Germany) to obtain the CD4+ T cells, following the instructions of the manufacturer.

This kit contains a Biotin-Antibody cocktail with antibodies against CD8, CD14, CD15, CD16, CD19, CD36, CD56, CD123, TCR γ/δ, and CD235a (Glycophorin A), and the inventors used it to label non-CD4+ cells, i.e., CD8+ T cells, monocytes, neutrophils, eosinophils, B cells, dendritic cells, NK cells, granulocytes, γ/δ T cells, or erythroid cells. Then, they used the second component of the kit, which is a CD4+ T Cell MicroBead Cocktail that consists of magnetic microbeads conjugated with monoclonal anti-biotin to target all non-CD4+ T cells. This suspension was added on an LS column that is able to retain the targeted cells. Finally, the inventors counted the resulting cells and analysed their purity by flow cytometry.

### 3.2. CD4+ T cell purity assay

To determine the CD4+ T cell purity, the inventors incubated the cells with antihuman CD3 FITC and antihuman CD4 PE (Immunotools GmbH, Germany) during 30 min at 0°C. Then, they washed the samples in PBS with 0.1% of FBS and analyzed them through flow cytometry. For experiments, the inventors only used samples that were at least 90% positive for both CD3+ and CD4+ (usually CD3+CD4+ T cells > 95%). Viability was constantly above 80% (usually viability > 90%).

### 3.3. CSFE staining and proliferation assay

To study the proliferation of CD4+ T cells, they were stained with a CellTrace CFSE cell proliferation kit provided by Thermo Fisher Scientific (USA), before seeding, following the instructions of the manufacturer.

### 3.4. Cell culture and seeding

The inventors seeded cells on 96 well plates (WP), except for the 3D printing experiments, which required 24WP given the characteristics of the printer (3D Discovery printer, RegenHU Biosystem Architects (Switzerland)). The inventors used the culture media Roswell Park Memorial Institute (RPMI) medium with 10% FBS and 1% penicillin/streptomycin. The inventors used a cell seeding concentration of a million cells/mL in all the cases, except for the proliferation studies with 3D printed scaffolds, when they used a concentration of 5·105 cells/mL. They induced the activation of cells by adding Dynabeads (Thermo Fisher, USA) in a 1:1 ratio, as suggested by the manufacturer. Positive controls were done by seeding the cells in suspension, as well as negative controls, which did not include Dynabeads. For the loading experiments with a positively charged immune molecule, the hydrogels were incubated with such a molecule (CCL21, CCL19 or ICAM-1) during 1 h. Afterwards, the supernatant was removed and the cells were seeded.

### 3.5. Immunostaining of CCL21 in glass surfaces functionalized with gold nanoparticles (Au NPs)

To prove that CCL21 binds to gold (Au), the inventors incubated recombinant CCL21 on the desired substrates in a PBS solution for 1 h at room temperature. The chosen substrates were glass surfaces half-functionalized with gold nanoparticles (Au NPs), with the objective of clearly see the difference between the part with and without Au in the same sample. After the incubation, the inventors washed the surfaces and performed a staining protocol. For that, the inventors incubated the substrates with a solution of primary antibody mouse anti-human CCL21 (Invitrogen, USA) in PBS for 1 h at room temperature, which was afterwards washed. After that, the inventors added a second antibody, goat anti-mouse Alexa 488 (Invitrogen, USA), which binds the first antibody and provides fluorescence, in a PBS with 1% of BSA solution. The incubation time was again of 1 h at room temperature under the dark. Finally, the inventors washed the samples imaged them with a fluorescence microscope (Olympus BX51, Japan).

### RESULTS

### 1. PEG-HEP HYDROGELS FOR CD4+ T CELL CULTURE

The inventors have studied the PEG-Hep hydrogels with the objective of mimicking the physicochemical properties of the SLOs, based on the properties of both PEG and Hep. On one hand, PEG is responsible to imitate the physical 3D structure of the LNs, due to its specific structural and mechanical properties, which can be easily regulated thanks to its synthetic nature. On the other hand, the heparin is resembling the function of the heparan sulphates naturally present in the ECM, acting as molecular sinks, storage sites, or presentation platforms to bind growth factors and chemokines. The inventors have fully characterized and used PEG-Hep hydrogels as 3D scaffolds for CD4+T cell activation, expansion, and differentiation, to study its further application into immunotherapy treatments.

More specifically, the inventors have synthetized, designed, and characterized PEG-Hep hydrogels with different stiffness, porosities, and loading capacities, in order to achieve the desired properties to mimic the ECM of SLOs, and have used said hydrogels for CD4+ T cell culture under different conditions, studying the resulting changes observed in proliferation and in the phenotypes achieved in comparison with the suspension cultures.

### 1.1. Synthetic PEG-Hep hydrogels

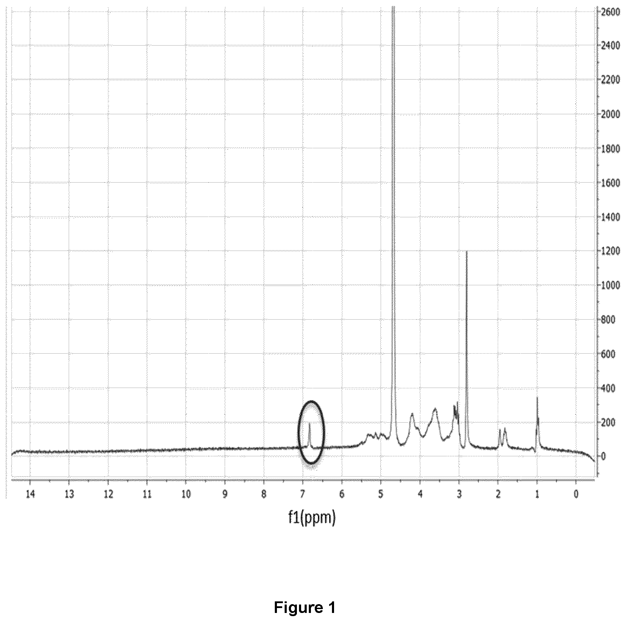

First, the inventors optimized the protocol of functionalization of heparin with maleimide by leaving the reaction overnight. The results were analyzed by proton nuclear magnetic resonance (H-RMN) spectroscopy (Figure 1). As shown in the spectrum, there is a peak at 6.83 ppm, corresponding to the protons of the maleimide linked to the heparin, which is slightly shifted from the reported peaks of the free maleimide ring at 6.86 ppm.

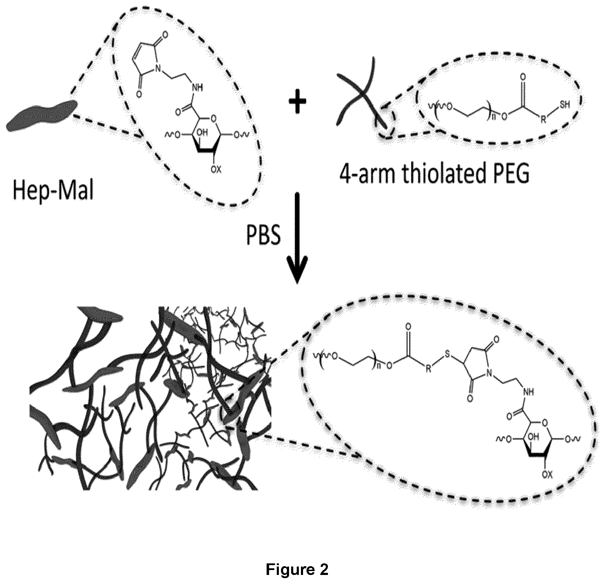

Once the heparin was fully functionalized, the inventors studied the hydrogel formation. For that, a solution of a 4-arm thiolated PEG (PEG-SH) was mixed with a solution of functionalized heparin in a proportion of 1:1.5, in PBS. As mentioned before, hydrogels with different mechanical properties can be obtained by varying the percentage of PEG. Thus, the inventors prepared hydrogels with the 3%wt, 4%wt, and 6%wt of PEG, all of them with the same proportion of PEG:Hep (1:1.5). PEG-Hep hydrogels were formed through a maleimide-thiol reaction between the maleimide of the functionalized heparin, and the thiols of the PEG, which results in a covalent crosslink and the consequent gelation (Figure 2).

### 1.2. Structural and mechanical properties of PEG-Hep hydrogels

To optimize the resulting hydrogels for immune cell culture, the inventors performed various experiments, and characterized their physical properties (e.g. stiffness, porosity, gelification time, and pore interconnectivity). Rheology was the technique used to characterize the gelification time and stiffness of the hydrogels. SEM was used to observe the structure of the hydrogel in the sections and surface. Finally, x-ray microtomography was used to obtain 3D images of the scaffold and study their pore interconnectivity.

### 1.2.1. Rheology

The main rheological technique to characterize hydrogels is small-amplitude oscillatory shear (SAOS). SAOS is used to identify the equilibrium shear modulus of a gel (Ge), which is the ratio of stress and strain in a balanced state where the material supports stress without deformation. To determine the Ge of a gel, the storage modulus (G') and the loss modulus (G") are measured in the LVE limit, and the limiting value of G' at low frequency is identified as the shear modulus.

This technique requires a trial-and-error approach to find the appropriate values of strain and frequency of each gel. Thus, the measurements require strain sweeps, frequency sweeps, and time sweeps at 37°C to calibrate the range of pressure and frequency where the hydrogels maintain their viscoelastic behavior. In strain sweeps, G' and G" are measured at a fixed frequency, while the pressure applied to the hydrogel increases within the range of interest. For frequency sweeps, the procedure is quite similar but, in this case, the pressure applied is constant and is the frequency which is changed. These two experiments show the values of pressure and frequency where the hydrogels maintain their viscoelastic behavior, and which are applied to the time sweep, where the changing variable is the time.

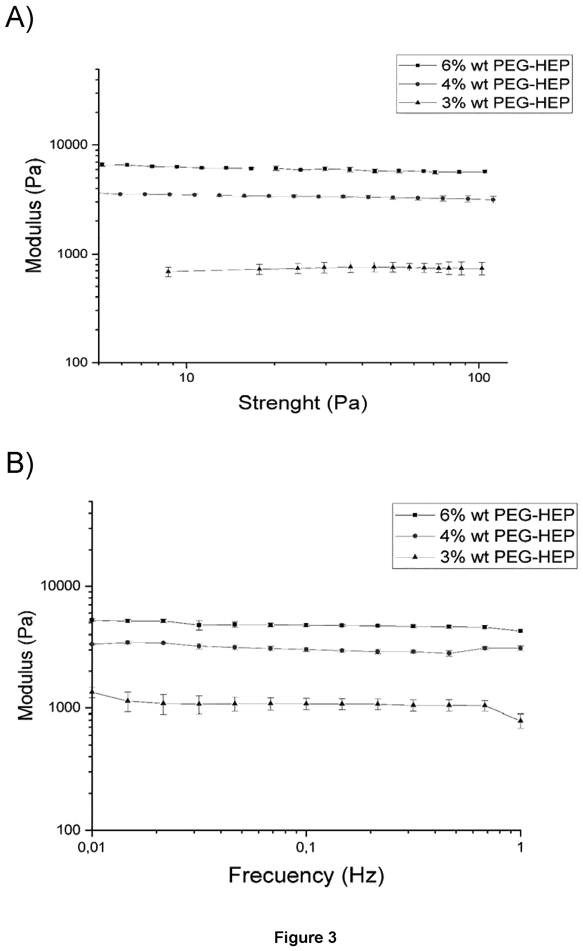

To perform these experiments, the inventors prepared hydrogels with different percentages of PEG (3%wt, 4%wt, and 6%wt). Strain sweeps were performed at 37°C and a constant frequency of 1.0 Hz, while the pressure was conducted from 1 Pa to 150 Pa on fully formed hydrogels (after optimizing the range where the hydrogels maintain their viscoelastic behavior). Then, frequency sweeps were performed from 0.01 Hz to 10 Hz at a constant strength of 50 Pa (Figure 3).

All hydrogels showed linear behavior of G' from a strength of 10 Pa to 100 Pa. More interestingly, samples of 3%wt show a G' lower than those of 4%wt and 6%wt, i.e. they can accumulate less energy in their structure without causing deformation. Thus, hydrogels with less amount of PEG are softer than those with higher amounts of PEG. The frequency sweeps were linear from 0.01Hz to 1Hz. Similarly, lower G' values were obtained for the 3%wt than the 4%wt and 6%wt PEG-Hep hydrogels. The values of the equilibrium shear modulus (Ge) achieved were 4.5±0.2 KPa for the 6%wt PEG-Hep hydrogel, 3.1±0.1 KPa in the case of 4%wt PEG-Hep, and 1.1±0.1 KPa for the 3%wt PEG-Hep. G" was too small to be measured for fully formed hydrogels. It is worth noting that these values of Ge correspond to fully formed and completely hydrated hydrogels. This is important because this is the state in which hydrogels were applied to cell culture.

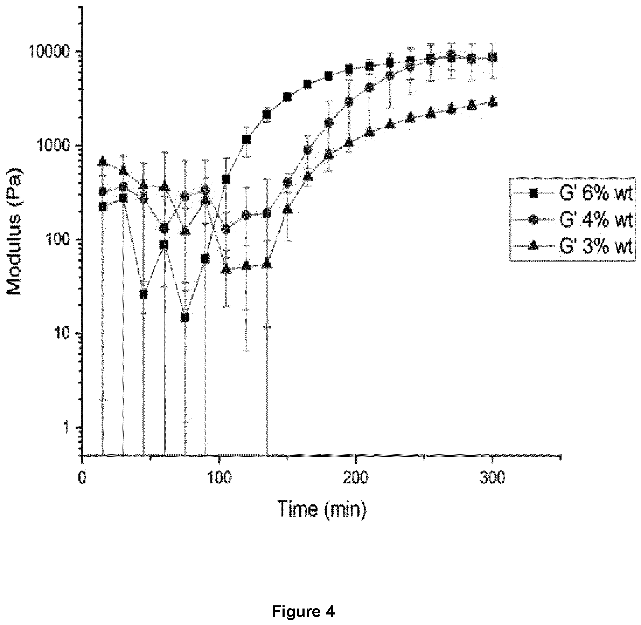

The inventors performed the time sweeps for the characterization of the gelification process, at 50 Pa and 0.1 Hz, values in which PEG-Hep hydrogels showed to maintain their viscoelastic behavior, with the rheometer at 37°C (Figure 4). The 6%wt and 4%wt PEG-Hep hydrogels were stabilized after ca. 200 min, at a storage modulus of 8.2±0.5 KPa, while the 3%wt PEG-Hep hydrogel required 240 min at a storage modulus of 2.7±0.2 KPa. The inventors analyzed the gelification process and the properties of the hydrogel when this process ends, without the addition of water or any other possible treatment. This means that, without the hydration process, the PEG-Hep hydrogels were more compact and stiff. Although this experiment was useful to characterize the gelification process, the stiffness values relevant for immune cell culture are the ones reported above, i.e. the ones obtained after hydrogel hydration in PBS.

### 1.2.2. SEM

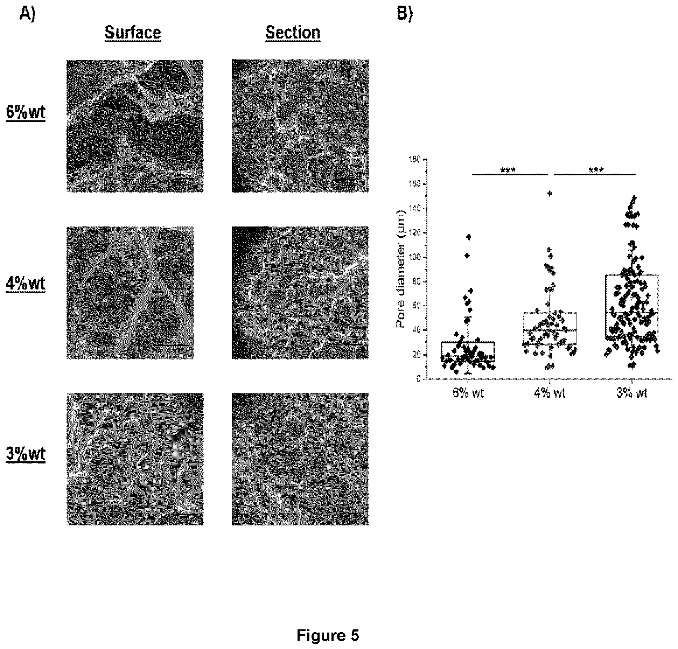

After the rheological characterization, the inventors studied the pore size of fully formed and hydrated PEGHep hydrogels at different percentages of PEG (6%wt, 4%wt, and 3%wt) by SEM imaging of their surface and section (Figure 5).

The median pore size of the 6%wt PEG-Hep hydrogels resulted of 20 µm with a porosity range of 5-50 µm, which increased to 40 µm for the 4%wt hydrogels with a range of 20-75 µm, and to 55 µm with a range of 25-105 µm for the 3%wt PEG-Hep hydrogels. These results show that the lower the amount of PEG present in the sample, the higher the porosity of the hydrogel. In order to apply PEG-Hep hydrogels to immune cell culture, the inventors chose the 3%wt PEG-Hep hydrogel due to its porosity and mechanical properties. For a deeper study of this hydrogel, the inventors measured the interconnectivity of its pores through 3D x-ray microtomography.

### 1.2.3. X-ray microtomography

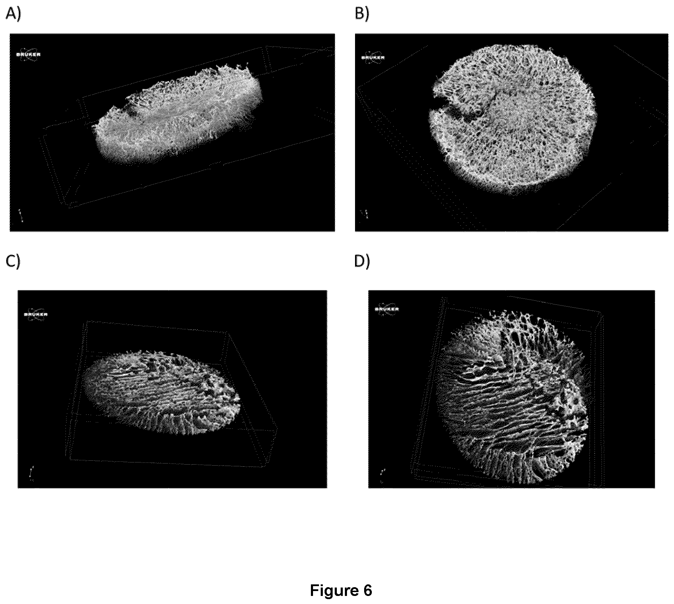

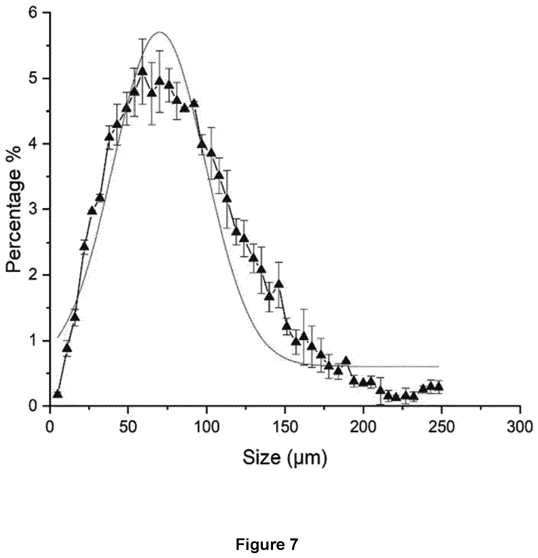

In addition to the previous characterization, the inventors freeze-dried the 3%wt PEG-Hep hydrogel and lyophilized it in order to obtain the dried 3D structure to characterize it by X-ray microtomography. The images achieved support the data obtained by SEM and provide high quality images and videos of the internal structure of the hydrogels where the interconnectivity of the pores can be seen (Figure 6A, B, C y D). Moreover, the percentage of the different pore sizes measured by this technique was represented and fitted to a Gaussian model (R2 = 0,998; Figure 7). From this analysis the inventors extracted that most of the pores measured around 70 µm, in agreement with the data obtained by SEM, where the median pore size observed was of 55 µm.

### 1.3. Biofunctionalization of PEG-Hep hydrogels: loading capacity

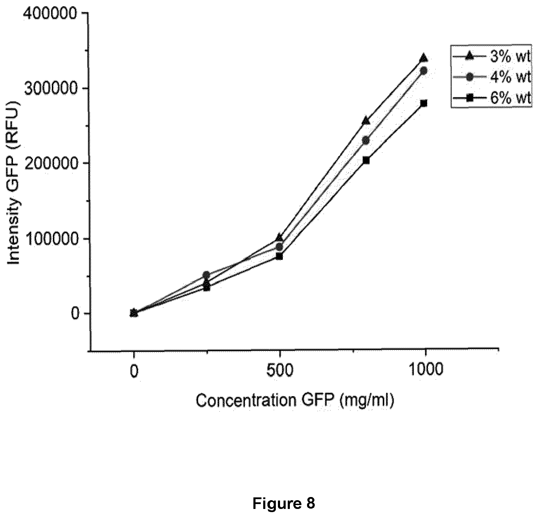

To confirm the ability of PEG-Hep hydrogels to attract positively charged molecules to their negatively charged heparin units, the inventors incubated different concentrations of GFP, which was used as a positively charged protein model (estimated charge of +5.6 mV), with the hydrogels during 1 h to observe the dependency of the loading capacity of the hydrogel with the concentration of positively charged protein. The fluorescence was maintained by the hydrogel after rinsing, indicating that the GFP was retained inside (Figure 8). The 3%wt of PEG-Hep hydrogel showed the highest retention of GFP, which suggest that these hydrogels have a better pore interconnectivity than the rest, so that the GFP solution can easily reach the whole hydrogel.

### 1.4. Unloaded PEG-Hep hydrogels applied to CD4+ T cell culture

Once the PEG-Hep hydrogels of different PEG percentages (6%wt, 4%wt, and 3%wt) were fully characterized, the inventors chose the 3%wt PEG-Hep hydrogels for CD4+ T cell culture, given their mechanical properties, higher porosity and interconnectivity as well as loading capacity. The inventors analyzed T cell proliferation through CFSE staining and flow cytometry. Thus, the inventors calculated the expansion, replication, and proliferation indexes 5 and/or 6 days after seeding. The expansion and replication indexes determine the fold-expansion of the overall culture and that of the responding cells, respectively, whereas the proliferation index is equal to the number of divisions that cells from the original population have undergone divided by the number of divided cells.

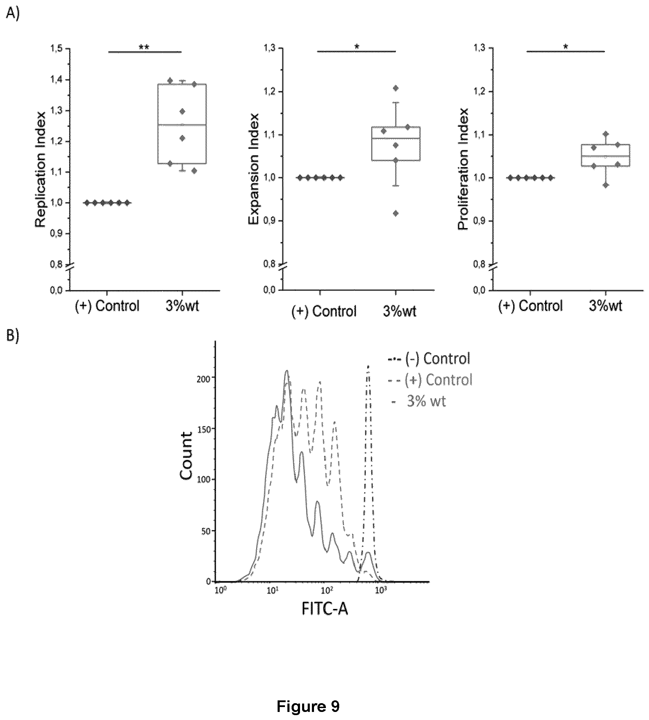

The inventors normalized results of the unloaded 3%wt PEG-Hep hydrogels used as a scaffold for CD4+ T cell culture to the positive control (Figure 9A). The median of the normalized replication index obtained was 1.25, i.e. an improvement of a 25% was achieved, whereas the expansion and proliferation indexes showed a median of 1.1 and 1.05, respectively. All three parameters showed statistically significant increases compared to the positive controls, which corresponded to cultures in suspension with Dynabeads. The strongest difference was observed for the replication index, which indicates that the responding cells that get activated in the synthetic hydrogels proliferate more than the activated cells in suspension. A representative graph of the peaks of fluorescence obtained in the flow cytometer after culturing is also shown (Figure 9B). This graph shows the displacement of the CFSE fluorescence peaks to the left in the positive control and sample compared to the negative control, indicating the new generations of cells obtained.

These results indicate, not only that PEG-Hep hydrogels do not show any cytotoxicity for the cells, but also that the 3D physical effect of the hydrogel, even without the introduction of any chemical stimuli, already causes an improvement in CD4+ T cell proliferation.

### 1.5. Study of different chemical stimuli to introduce into PEG-Hep hydrogels

Once the beneficial effect of unloaded PEG-Hep hydrogels was observed, the inventors studied the effect of different chemical stimuli through the introduction of biomolecules into the hydrogels. For the introduction of such chemical stimuli, the inventors used the already proved capacity of the heparin present in the hydrogels for anchoring positively charged molecules by electrostatically interactions, mimicking the natural function of the heparin sulphates present in the ECM of the LNs.

The inventors studied the positively charged molecules associated with immune cell activation and expansion in suspension and fixed in 2D and 3D systems to observe their effect on the proliferation and differentiation of CD4+ T cells. The inventors chose the cytokines CCL21 and CCL19 as well as the cell adhesion molecule ICAM-1.

### 1.5.1. CCL21

Chemokine (C-C motif) ligand 21 (CCL21) is a small cytokine involved in the activation process of the immune system. It plays an important role in costimulating the expansion of CD4+ and CD8+ T cells and inducing Th1 polarization. It is highly expressed in the endothelium of lymphatic vessels and SLOs and interacts with T cells and mature DCs which express the chemokine receptor CCR7.

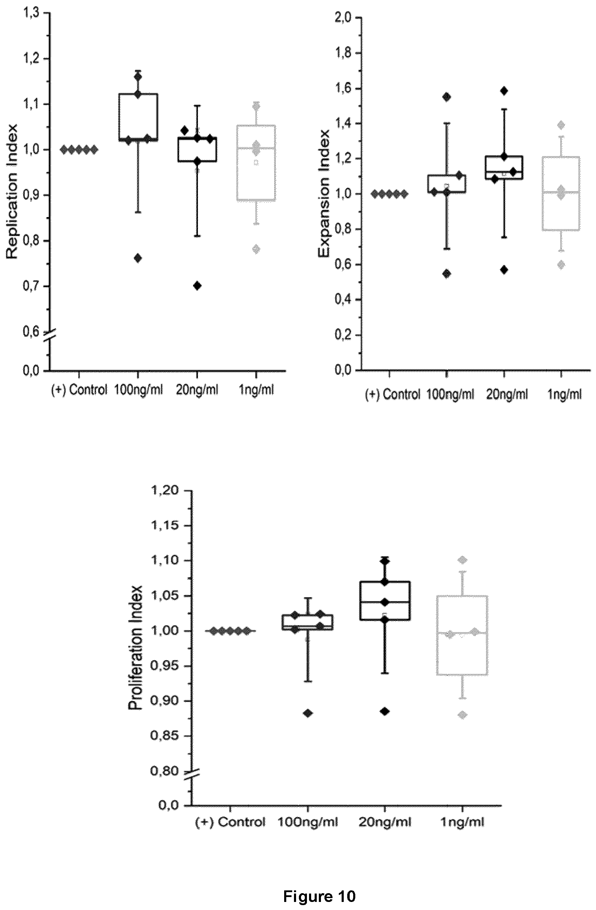

Initially, the inventors chose the concentrations 1 ng/mL, 20 ng/mL, and 100 ng/mL for experiments in suspension (Figure 10). The median values for the replication index for 100 ng/mL, 20 ng/mL, and 1 ng/mL were 1.01, 0.95, and 0.96, respectively, i.e. very similar to the positive controls. The inventors observed the same tendency for the expansion index, with median values of 0.99, 1.02, and 0.99, and the proliferation index with values of 1.05, 1.12, and 1.00 for 100 ng/mL, 20 ng/mL, and 1n g/mL, respectively. No significant differences could be observed among the different CCL21 concentrations in suspension.

In the next steps, the inventors tested the capacity of CCL21 to effect T cell proliferation when immobilized. To analyze the effect of such immobilization in a well-defined system, they used planar Au surfaces.

With this objective, they first corroborated the capacity of immobilizing the cytokine CCL21 through its cysteine residues. For that, the inventors used a surface that was functionalized with a quasi-hexagonal pattern of AuNPs only on its lower part by dip-coating. The inventors used block copolymer micellar lithography (BCML) to prepare a Au-loaded micellar solution by dissolving an amphiphilic block copolymer in an apolar solvent to create reverse micelles. The inventors dipped-coated commercial ITO-coated glass substrates with the loaded Au micellar solution obtaining AuNPs with a lateral interparticle distance of 68 ± 20 nm (Figure 11B).

The inventors passivated these surfaces with PEG overnight and incubated with CCL21 during 1 h. After the incubation, they performed an immunostaining (Figure 11C) using human anti-CCL21 as primary antibody and mouse anti-human Alexa 488 as secondary antibody to observe through fluorescence where was the cytokine retained. A scheme of the experiment performed can be seen in Figure 11A, wherein fluorescence could only be detected in the part of the surfaces decorated with AuNPs, proving that CCL21 was fixed on their surface.

After verifying that CCL21 binds to Au, the inventors prepared 2D Au surfaces functionalized with CCL21, and used them for cell culture. They tested different concentrations of CCL21 to find the optimum amount of cytokine needed to obtain CD4+ T cell proliferation of relevant phenotypes. Elevated concentrations of CCL21 were avoided given their potentially inhibitory effect.

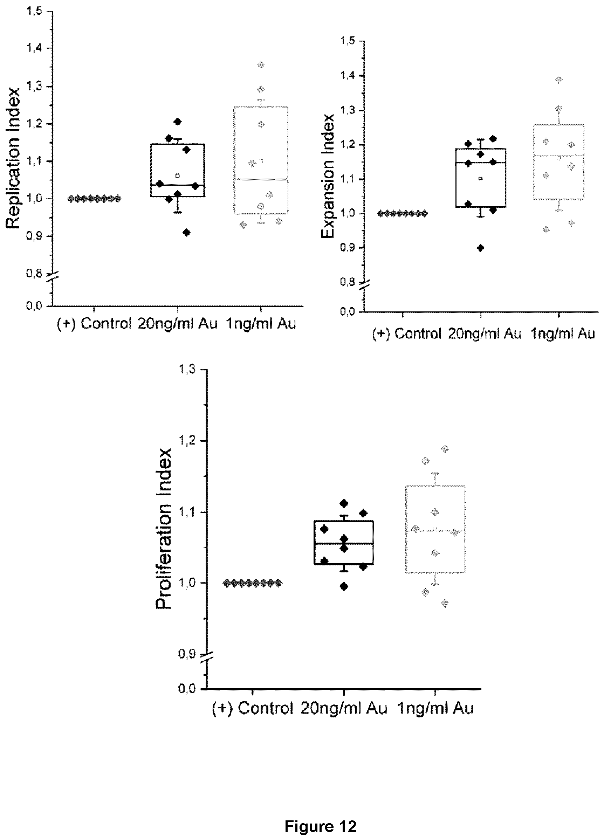

Thus, the inventors immobilized CCL21 on Au surfaces at concentrations of 1 ng/mL and 20 ng/mL (Figure 12), which resulted in significant changes (**p < 0.01) for the proliferation and expansion indexes. Specifically, the median values for the expansion index were of 1.1 and 1.16 for the concentrations of 20 ng/mL and 1 ng/mL respectively, showing an improvement of 10% and 16% in comparison with the positive control. Similarly, the proliferation index increased to 1.06 and 1.08 for each concentration. The replication index also showed a slight tendency to increase with median values of 1.06 for 20 ng/mL and 1.1 for 1 ng/mL.

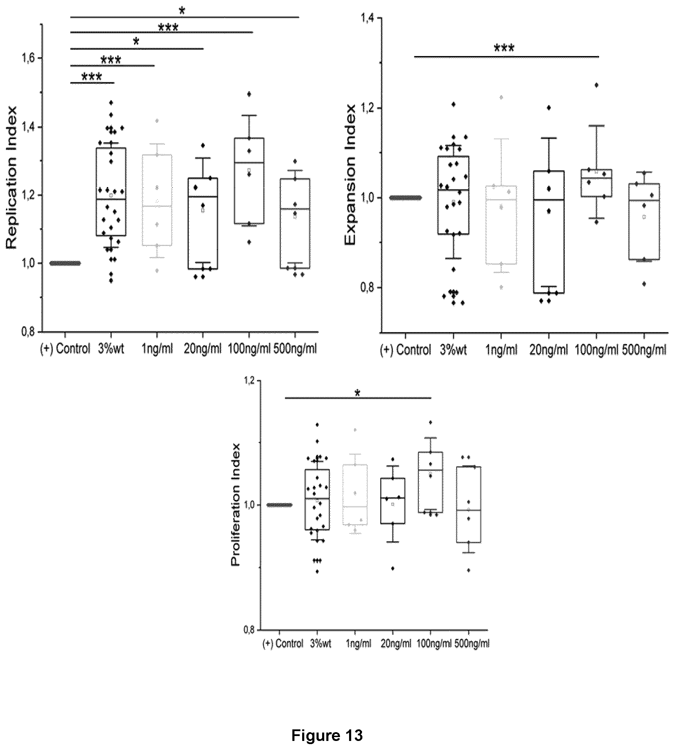

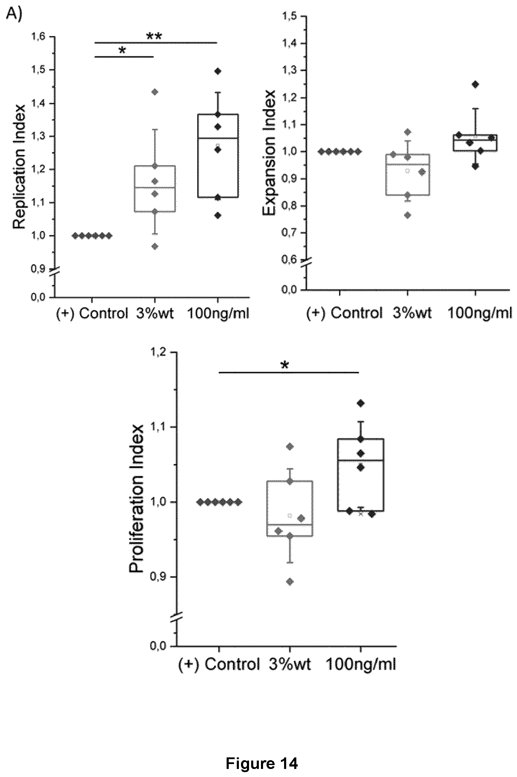

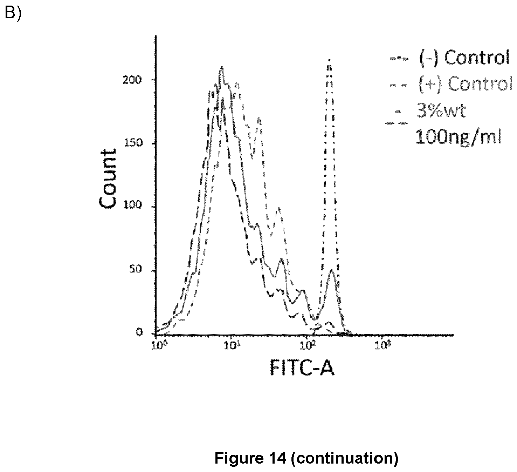

These results confirmed that CCL21 increases CD4+ T cell proliferation when fixed. Accordingly, the inventors loaded this cytokine into PEG-Hep hydrogels. The inventors incubated different concentrations of CCL21 in PEG-Hep hydrogels and identified 100 ng/mL as the concentration which led to the highest expansion results in contrast with the CCL21-immobilized on Au surfaces (Figure 13). This difference was probably caused by the increase in total area available when moving from 2D to 3D biomaterials. As shown in Figure 14, all the proliferation indexes were improved when the hydrogel was loaded with CCL21 in comparison with the unloaded hydrogel. For the hydrogels with 100 ng/mL of CCL21, a 30% increase in the replication index was obtained in comparison with the positive control (1.3 of median value), while the unloaded hydrogel showed a 15% (1.15 of median value) of improvement. The proliferation and expansion indexes showed less pronounced increases with average median values of 1.05 and 1.06, respectively (Figure 14A). Again, the strongest difference was observed for the replication index. A representative graph of the peaks of fluorescence obtained in the flow cytometer after culturing is also shown (Figure 14B).

Once verified that PEG-Hep hydrogels functionalized with CCL21 increase CD4+ T cell proliferation, the inventors performed differentiation assays 5 days after seeding to determine the phenotype of the resulting T cells. The changes in the surface of CD4+ T cells caused by their activation result in different phenotypes, which were analyzed by flow cytometry. These phenotypes were naïve (TN; CD45RO-/CD62L+), central memory (TCM; CD45RO+/CD62L+), and effector memory (TEM; CD45RO+/CD62L-).

CD45 is a transmembrane protein tyrosine phosphatase (receptor type C) expressed on the cell surface of human leukocytes. The isoforms of CD45 are associated to different differentiation stages and are commonly used as markers to identify different types of immune cells. More specifically, CD45RA is associated with cells that have not encounter yet a matching antigen, and they are therefore naive. In contrast, once a naive cell gets activated, it expresses the isoform CD45RO, which is preserved in memory T cells. Consequently, TN are CD45RA+ and CD45RO-, whereas TEM are CD45RA- and CD45RO+. The TCM are a special type of cells, which have already encounter a matching antigen and thus are CD45RO+, but preserve a high potential of reproduction. These cells are in an "intermediate" differentiation state and some studies have suggested their higher potential to eliminate tumors compared to other cell types. This capacity has been explained by their higher capacity to proliferate than memory cells, i.e. the presence of more immune cells per cancer cell, and their higher specificity than naive cells, which are cells that have also a high proliferation capacity.

CD62L, also named L-selectin is a type I transmembrane cell adhesion molecule expressed on most circulating leukocytes, including neutrophils. L-selectin is one of three family members: L-, E- and P-selectin. Each selectin is defined according to the cell type in which it was first characterized (L = lymphocyte, E = endothelial cell, P = platelet). TN cells express CD62L because they need to enter SLOs to encounter their antigen. TCM, which have already encountered antigen are CD62L+ because they still need to localize in SLOs, where they reside ready to proliferate upon re-encountering their specific antigen. TEM do not express CD62L, as they circulate in the periphery and have immediate effector functions upon re-encountering their specific antigen.

The percentages of CD4+ T cells that express CD45RO and CD62L prior to stimulation (negative control) are submitted to the intrinsic donor variability. They mostly showed a TN phenotype with a median value of 53%, whereas the TEM and TCM phenotypes were found in lower percentages, 12% and 32%, respectively.

After stimulation, the median value of TN cells decreased to 4% in suspension (positive control). Both unloaded and loaded (100 ng/mL of CCL21) hydrogels exhibited a decrease of this phenotype, being the median values 14% and 11% respectively. Consequently, the TEM and TCM phenotypes increased in comparison with the negative control. Specifically, the median values for the TCM phenotype of the positive control, unloaded, and loaded hydrogels were 68%, 51%, and 47%, respectively. For the TEM phenotype, the median values raised to 26% for T cells in suspension, 21% for unloaded hydrogels, and 33% for hydrogels with 100 ng/mL of CCL21, showing an increase of effector cells for those seeded in cytokine loaded hydrogels. These results point out that PEG-Hep hydrogels can be used to modify the resulting phenotype of T cells and different chemical inputs can be used to achieve diverse differentiation pathways.

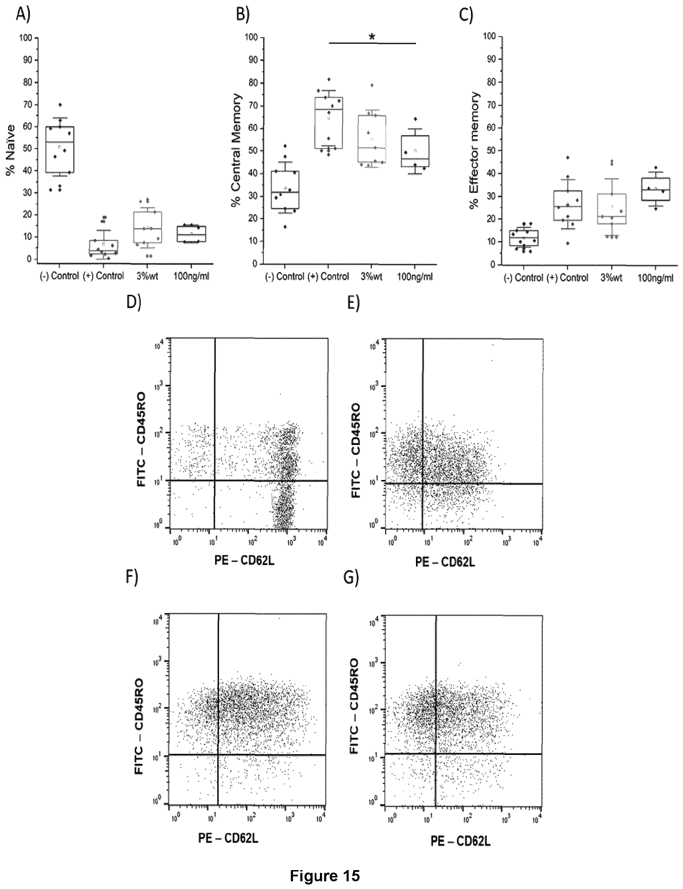

Finally, representative dot plots of the negative control, positive control, unloaded and loaded hydrogels are shown (Figure 15D-G), wherein the resulting activated populations of cells evolve differently in suspension and in the hydrogel, although both come from the same initial population of cells.

After analyzing these results, the inventors concluded that CCL21 improves CD4+ T cell proliferation and tune differentiation, increasing the total amount of effector T cells.

### 1.5.2. CCL19

CCL19 is a cytokine of the same family of CCL21, which acts as a potent inducer of T cell proliferation in DC - T cell co-cultures although only with activated DCs. This cytokine interacts with the CCR7 receptor such as CCL21. However, slight conformational changes in CCR7 following binding of the two different chemokines results in differential T cell signaling.

The inventors used the same concentrations for CCL19 than the previously employed in solution for CCL21, 100 ng/mL, 20 ng/mL, and 1 ng/mL. After 6 days of culture they measured the proliferation results. In this case the highest increase of the proliferation parameters was observed for the concentration of 1 ng/mL, with median values of 1.19, 1.20, and 1.06 for the replication, expansion, and proliferation indexes, respectively.

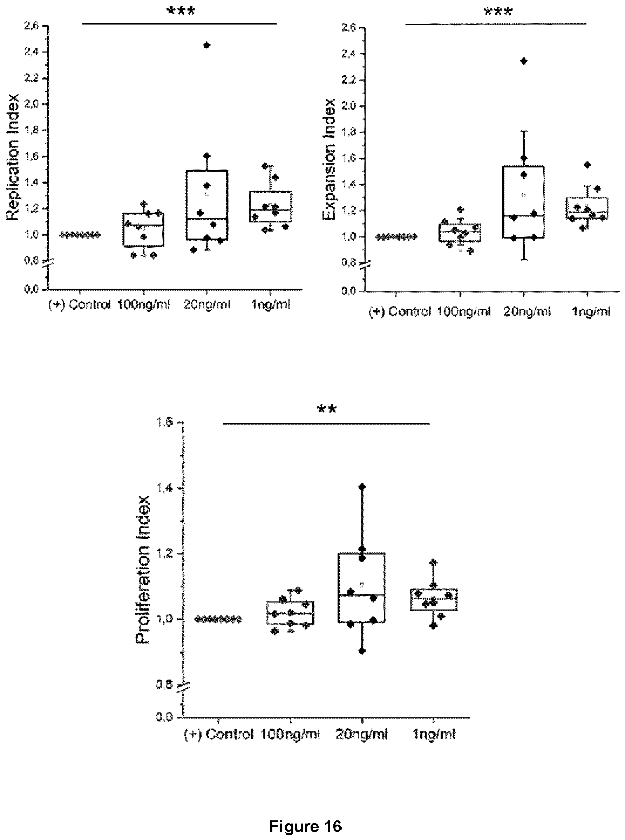

Although no statistical changes were observed for the concentrations of 100 ng/mL and 20 ng/mL of CCL19 in suspension, the inventors observed a tendency to increase the proliferation of CD4+ T cells when a concentration of 1 ng/mL was employed. In contrast with the results observed for CCL21, where no significant differences were observed, CCL19 as an obligate soluble chemokine that it is, showed its influence in suspension for the concentration of 1 ng/mL (Figure 16).

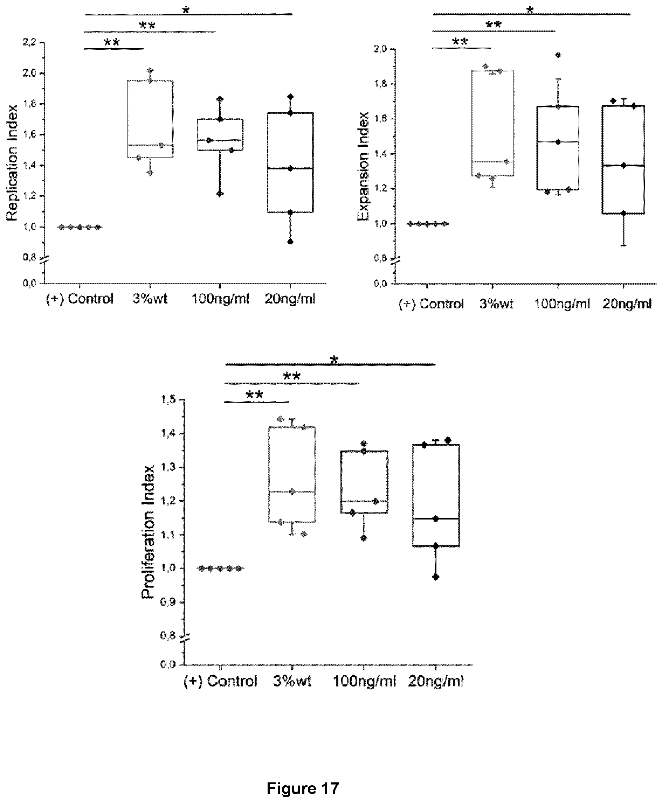

Subsequently, the inventors loaded different concentrations of CCL19 in PEG-Hep hydrogels to study the differences between having CCL19 in solution and anchored to the hydrogels (Figure 17). All the proliferation indexes showed an improved tendency in comparison with the positive control. The replication index showed an increase of 50% and 56% for unloaded hydrogels and loaded with 100 ng/mL of CCL19, respectively (1.5 and 1.53 of median value). A lower increase of 38% for hydrogels loaded with 20 ng/mL of CCL19 was obtained. A similar tendency was observed for the expansion index, with median values of 1.35, 1.47, and 1.34 for unloaded, 100 ng/mL and 20 ng/mL of CCL19 respectively. Finally, the achieved proliferation indexes median values were 1.23, 1.20, and 1.14 respectively.

### 1.5.3. CCL21 and CCL19

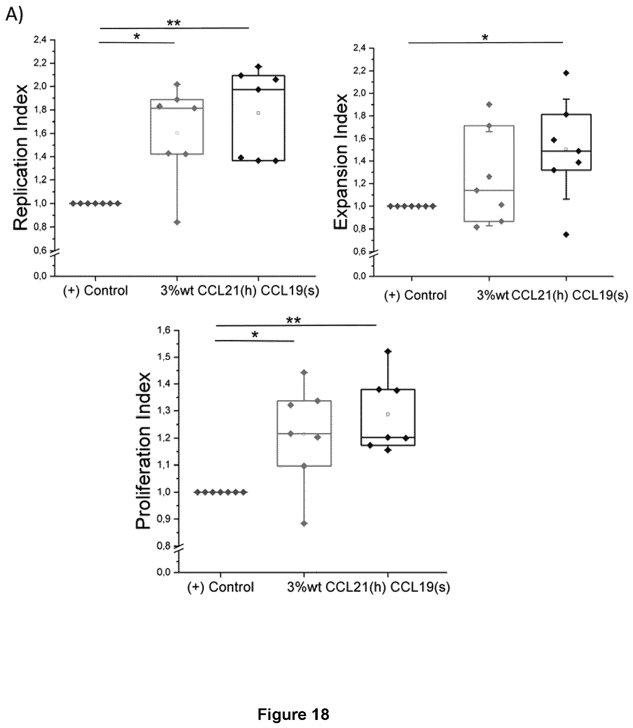

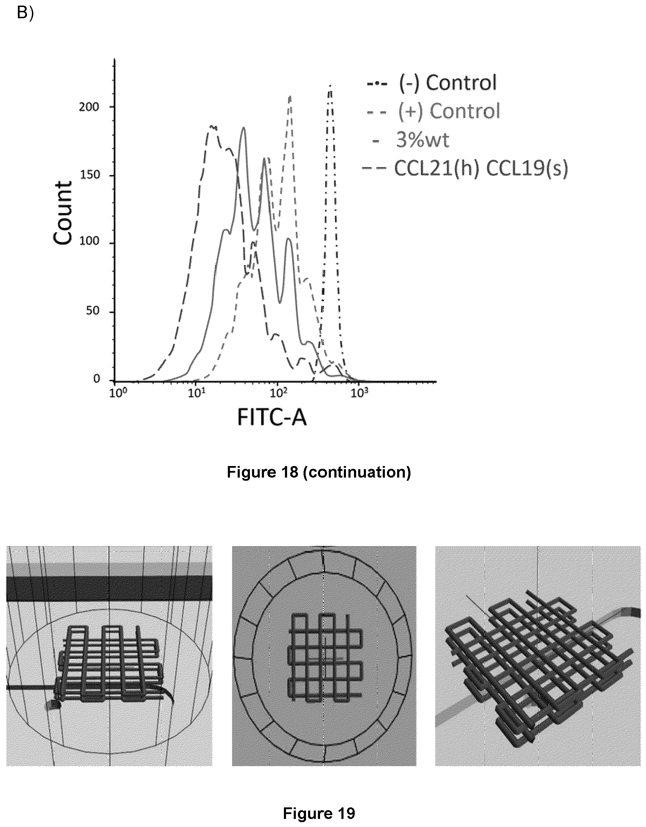

Once having studied CCL21 and CCL19 separately, the inventors used both cytokines with the objective of mimicking the natural environment of the LNs and maximizing the proliferation results. In this case, they loaded CCL21 in the hydrogel during 1 h and added CCL19 in solution with the media [CCL21(h) CCL19(s)], both with concentrations of 100 ng/mL. The inventors measured proliferation 6 days after seeding (Figure 18).

These hydrogels with both cytokines duplicated the replication index (2.00 of median value) of the positive control and showed also an improve compared to the unloaded hydrogel, which had a median value of 1.80. The expansion index was also improved, obtaining median values of 1.50 for the hydrogel with cytokines and 1.13 for the unloaded hydrogel. Finally, the proliferation index showed similar median values, 1.21 and 1.20 respectively, although higher statistical difference was observed when both cytokines were employed (Figure 18A). A representative graph of the peaks of fluorescence obtained in the flow cytometer after culturing is also shown (Figure 18B). These results show the benefits of mimicking the natural environment of cells with both cytokines, which resulted in higher proliferation rates than the hydrogels with only one of the cytokines or none.

### 1.5.4. ICAM-1

Finally, the inventors used a different type of positively charged immune molecule, the cell adhesion molecule ICAM-1, a key molecule in immune-mediated and inflammatory processes functioning as an important co-stimulatory signal for the activation of T cells.

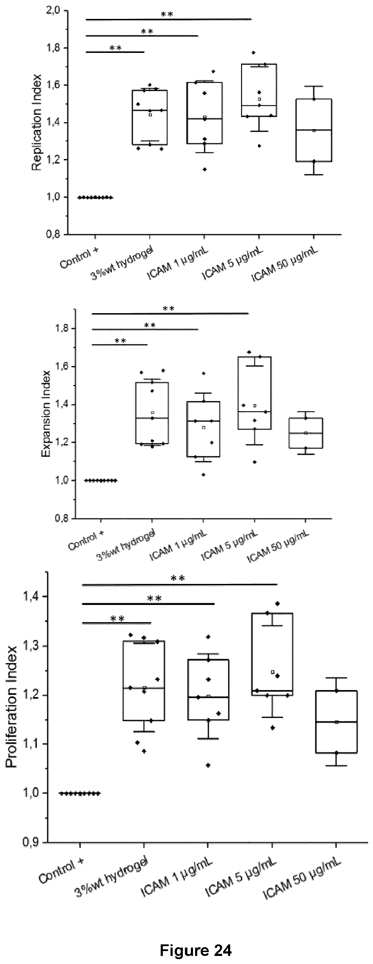

The inventors used different concentrations of ICAM-1, 1 µL/mL, 5 µL/mL and 50 µL/mL, to load the hydrogels before cell seeding (Figure 24). After 6 days, the proliferation index showed the median values of 1.22, 1.20, 1.25 and 1.15, whereas the expansion index exhibited median values of 1.36, 1.28, 1.40 and 1.25 for the unloaded 3% wt hydrogel as well as ICAM-1 (1 µL/mL), ICAM-1 (5 µL/mL), and ICAM-1 (50 µL/mL) loaded hydrogels, respectively. Finally, median values of 1.44, 1.43, 1.53 and 1.36 were observed for the unloaded 3% wt hydrogel and ICAM-1 (1 µL/mL), ICAM-1 (5 µL/mL) and ICAM-1 (50 µL/mL) loaded hydrogels, respectively.

Although the use of a hydrogel improves the state of the art expansion system (Dynabeads in suspension), no statistical changes were observed for the concentrations of 1 µL/mL, 5 µL/mL, and 50 µL/mL of ICAM-1 in suspension. Nevertheless, the inventors observed a slight increase of 2.3% for the proliferation index, 2.9% for the expansion index, and 6.1% for the replication index, when compared with the unloaded 3%wt hydrogel. These results might be explained by the lower estimated net charge of ICAM-1 (+4.8) compared to the one of CCL21 (+17.0), which will have a weaker interaction with the hydrogel that could result in a less efficient cellular response.

### 1.6. Conclusions

PEG-Hep hydrogels were synthesized, optimized, characterized, and loaded with different biomolecules to mimic the LNs. Hydrogels of different percentages of PEG were efficiently developed (6%wt, 4%wt, and 3%wt). The higher the PEG concentration, the higher the stiffness, the smaller the pore size and the interconnectivity. 3%wt PEG-Hep hydrogels showed an increase in the proliferation of CD4+ T cells and an influence on the resulting phenotypes, even without the addition of any chemical stimuli. CCL21, a positively charged immune molecule of the cytokine family, further induced CD4+ T cell proliferation when anchored to the PEG-Hep hydrogels. The highest proliferation parameters were achieved through the combination of two cytokines, CCL21 loaded to the hydrogels and CCL19 added in solution, mimicking the LNs. Moreover, the cell adhesion molecule ICAM-1 was also evaluated to demonstrate the versatility of the platform to introduce different types of positively charged immune molecules. This system could be further improved to fabricate artificial LNs, which are expected to overcome the limitations of current immunotherapies such as producing large amounts of T cells with therapeutic phenotypes.

### 2. PEG-HEP HYDROGELS FOR 3D PRINTING

3D printing is a technique that consists of producing 3D objects with precisely designed geometries in a layer by layer approach that can be used in biomedicine. For example, cell-laden 3D constructs can be built, which include cells as printable materials, to be used for implants in regenerative medicine or artificial tissues can be created with the objective of replicating the structures of native tissues.

Due to the beneficial effects showed by PEG-Hep hydrogels for T cell expansion above mentioned, the inventors analyzed these hydrogels as bioink for 3D printing, by optimizing the gelification process of PEG-Hep hydrogels and adjusting to the 3D printer requirements.

### 2.1. PEG-Hep hydrogels as bioink for 3D printing

To use the PEG-Hep hydrogels as bioink, the inventors optimized the gelification process to obtain well defined pre-designed 3D scaffolds for cell culture in a 3D Discovery printer from RegenHU Biosystem Architects (Switzerland). The inventors chose a design consisting of a grid with a separation of 1.5 mm between its lines and of 4 or 6 layers of height (Figure 19).

With this purpose, the inventors prepared 3%wt PEG-Hep pre-hydrogels in PBS by mixing the solutions with both reagents (PEG and Hep) and heated them up to 37°C. Afterwards, the resulting mixture was analyzed as bioink for 3D printing at different times. The inventors could performed the first printings after 3.5 h (after 3 h, the mixture was still too liquid), when the sample had enough consistency.

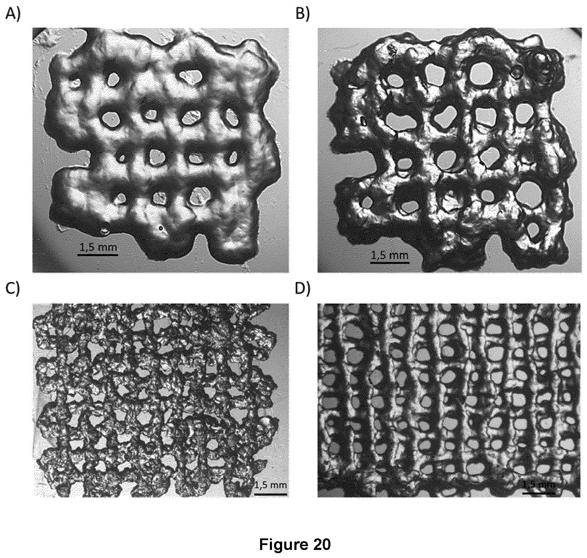

The resulting scaffolds had though a very low rigidity and no differentiated lines were achieved in the printed grid (Figure 20A). To improve that, samples were stored overnight under two conditions, at room temperature and in the incubator at 37°C, and the experiment was repeated after 24 h of gelation. The material stored at room temperature showed optimal properties for its printing, obtaining quite well defined scaffolds (Figure 20B). Nevertheless, samples stored at 37°C got very dried, making the printing heterogeneous and difficult, and the resulting scaffolds were not adequate for cell culture (Figure 20C). Additionally, different pressures of extrusion and tips for the printing were tested. After the corresponding optimization experiments, it was found that the optimum extrusion pressure was of 1.2 bar by using a conic tip with an inner diameter of 27 G (0.3606 mm).

Moreover, the inventors prepared PEG-Hep hydrogels for cell-laden experiments. Unexpectedly, the gelation was immediate when cell medium was used and the resulting gel could be properly printed (Figure 20D). Thus, cells can be introduced inside the hydrogel through printing.

### 2.2. PEG-Hep printed scaffolds for CD4+ T cell expansion

Once the inventors demonstrated that 3%wt PEG-Hep hydrogels can be used as bioink and the protocol was optimized, they printed 3D layered structures to observe their effect on CD4+ T cell culturing and ensure their (bio)compatibility. The inventors printed scaffolds of 4 and 6 layers in WP.

The inventors first evaluated unloaded printed hydrogels and after they incubated the scaffolds with 100 ng/mL of CCL21. Specifically, they cultured unloaded printed scaffolds of 4 and 6 layers with CD4+ T cells during 6 days and afterwards, they measured the replication, expansion, and proliferation indexes by flow cytometry and compared with the ones obtained for cells seeded in suspension (positive control). The inventors normalized the results to the positive control.

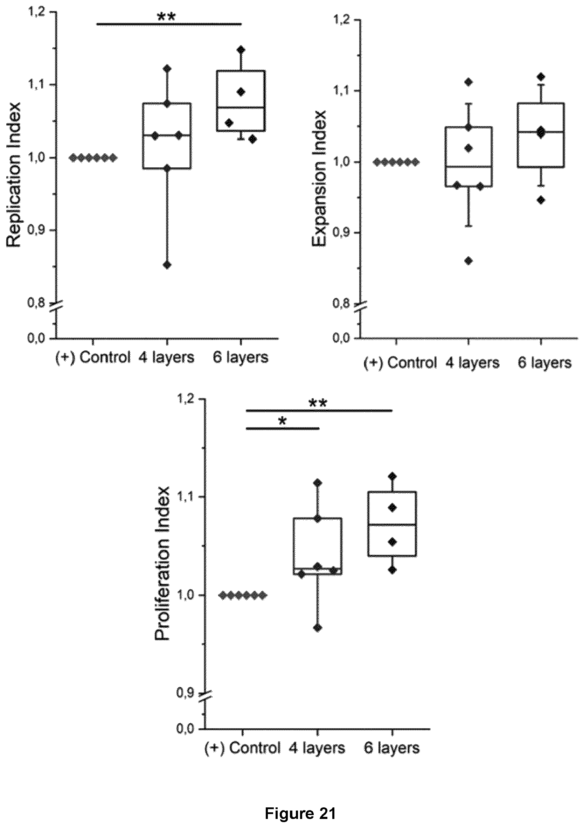

The PEG-Hep scaffold printed with a height of 4 layers exhibited a slight tendency to increase the proliferation parameters in comparison with the positive control, obtaining normalized values of 1.03 for the replication and proliferation indexes. The only significant change obtained was in the proliferation index. On the other hand, the PEG-Hep scaffolds printed with 6 layers of height showed stronger proliferation improvements. Specifically, the replication and proliferation indexes increased a 7% (1.07 as a result of their normalized value) and the expansion index a 4% (Figure 21). It can therefore be concluded that the presence of a printed 3D scaffold improves T cell proliferation and the higher the structure, the more the cells reproduce.

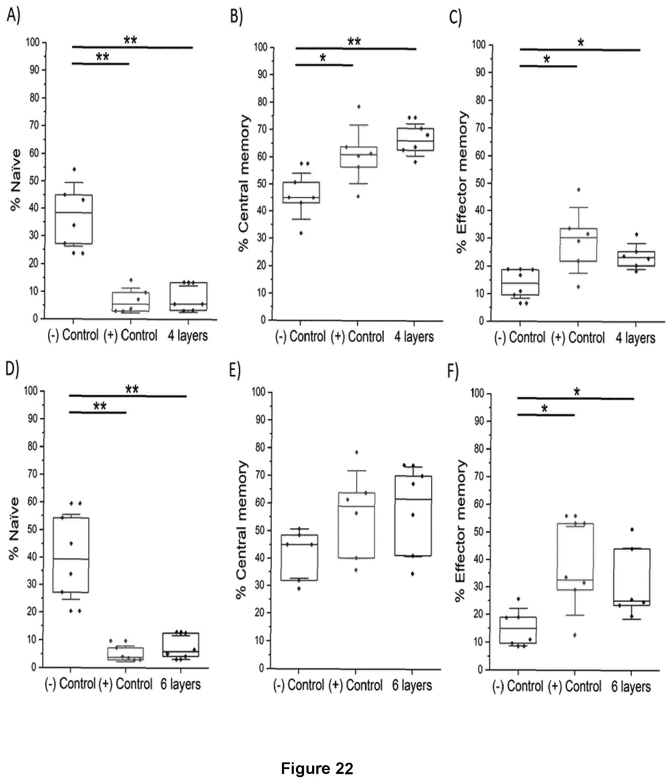

To determine if the phenotype of the resulting T cells was affected, the inventors performed a differentiation assay with scaffolds of 4 and 6 layers. The resulting CD4+ T cell populations were classified in naïve (TN; CD45RO- / CD62L+), central memory (TCM; CD45RO+/CD62L+), and effector memory (TEM; CD45RO+/CD62L-) 5 days after seeding (Figure 22).

In the 4 layers scaffolds (Figure 22A-C), the inventors observed a statistically significant increase of the percentage of TCM, which is a phenotype that has been associated with succesful clinical outcomes. Namely, the median value of TCM raised from the 45% of the negative control and the 61% of the positive control to the 66% for the 4 layer printed hydrogels (Figura 22B). On the other hand, the TEM median values augmented for CD4+ T cells activated in suspension from a 14% of the negative control to a 30%, while this increase was lower for cells seeded in the printed hydrogels, with a median value of 23% (Figure 22C). Naive cells decreased from a 39% of the inactivated cells to a 5% and 6% of cells activated in suspension and in hydrogels, respectively (Figure 22A). However, the inventors observed no significant changes between cells activated in suspension or using a scaffold in this population. The 6 layer scaffolds showed the same tendency. Specifically, the TCM (Figure 22E) and TEM (Figure 22F) phenotypes increased, while the TN decreased (Figure 22D). However, but the achieved differences were less pronounced, especially for the TCM.

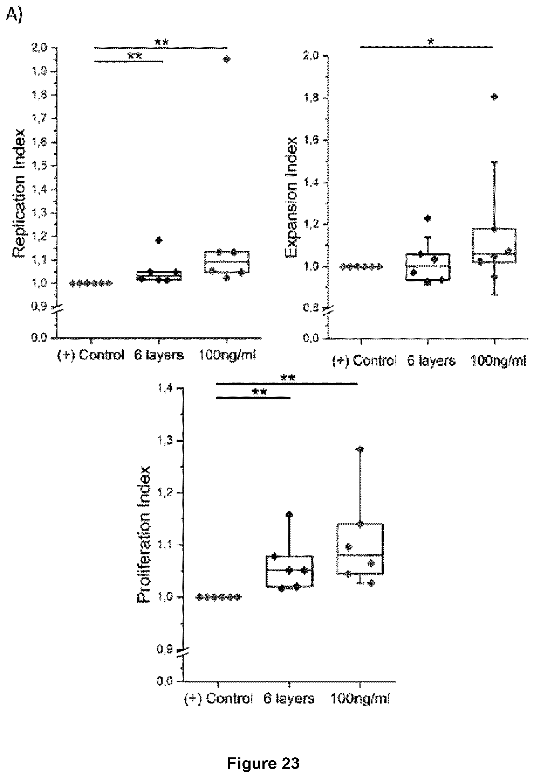

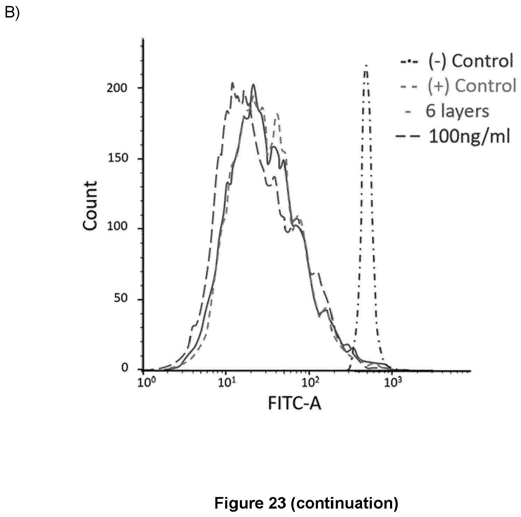

As previously done for non-printed hydrogels, the inventors incubated a solution of 100 ng/mL of CCL21 during 1 h prior to cell seeding to decorate the printed scaffolds and study its effect on CD4+ T cell proliferation (Figure 23). The replication index improved a 3% and 10% (with median values of 1.03 and 1.10) when using unloaded and loaded printed hydrogels, respectively, in comparison with the positive control. The expansion index showed no significant changes for the unloaded gels, slightly increasing its median value from 1.00 to 1.06 with the addition of the cytokine. Finally, the same tendency was obtained for the proliferation index with median values of 1.05 and 1.08 for unloaded and loaded hydrogels, respectively (Figure 23A). The CSFE diagram (Figure 23B) shows representative plots for each sample. Once again, all the proliferation indexes improved when using the hydrogel loaded with CCL21 in comparison with the unloaded hydrogel and the positive control.

### 2.3. Conclusions

After an adequate optimization of the gelification process, 3%wt PEG-Hep hydrogels can be used as an ink for 3D printing. Thus, 3D scaffolds were obtained with PEG and Hep diluted in both, PBS and media, opening the way to a wide range of applications, including cell-laden experiments. The proliferation of CD4+ T cells increased when cells were incubated in printed scaffolds, observing higher rates for scaffolds of 6 layers in comparison with 4 layer scaffolds, as expected by the presence of a larger amount of material. Additionally, these scaffolds also resulted in an increase of the percentage of TCM cells obtained after 5 days of incubation, which is known to be phenotype associated with effectiveness in immunotherapies. Finally, it is also worth mentioning that the addition of CCL21 as a chemical stimulus to the printed hydrogels, resulted in an increased proliferation of CD4+ T cells as observed for non-printed hydrogels.

## Claims

1. A hydrogel comprising a functionalized PEG multi-arm star polymer covalently combined with heparin, further comprising at least one positively charged immune molecule.

2. The hydrogel according to claim 1 wherein the functionalized PEG multi-arm star polymer is a functionalized PEG four-arm star polymer.

3. The hydrogel according to any preceding claim wherein the functionalized PEG multi-arm star polymer is a thiol-PEG multi-arm star polymer.

4. The hydrogel according to any preceding claim wherein the functionalized PEG multi-arm star polymer is a thiol-PEG four-arm star polymer of formula:

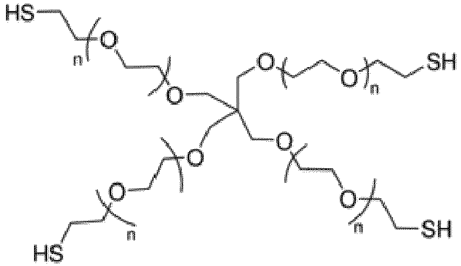

5. The hydrogel according to any preceding claim wherein the concentration of the functionalized PEG multi-arm star polymer ranges between 2%wt to 10%wt on the total weight percentage of hydrogel.

6. The hydrogel according to any preceding claim wherein the heparin is low molecular weight heparin.

7. The hydrogel according to claim 6 wherein the heparin is maleimide-low molecular weight heparin.

8. The hydrogel has a median pore size between 5 to 105 µm, preferably is 55 µm.

9. The hydrogel according to any preceding claims wherein the positively charged immune molecule is a cytokine and/or a cell adhesion molecule.

10. The hydrogel according to claim 9 wherein the cytokine is a chemokine selected from the list consisting of: CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL23, CCL24, CCL25, CCL26, CCL27, CCL28 and any combination thereof.

11. The hydrogel according to claim 10 wherein the chemokine is CCL21 and/or CCL19.

12. The hydrogel according to any one of the claims 9 to 11 wherein the concentration of cytokine ranges between 0.1 ng/mL to 250 ng/mL, preferably is 100 ng/mL.

13. The hydrogel according to claim 9 wherein the cell adhesion molecule is a intercellular adhesion molecule selected from the list consisting of: ICAM-1, ICAM-2, ICAM-3, ICAM-4, ICAM-5 and any combination thereof.

14. The hydrogel according to claim 13 wherein the cell adhesion molecule is ICAM-1.

15. The hydrogel according to claims 13 or 14 wherein the concentration of cell adhesion molecule ranges between 1 µg/mL and 50 µg/mL, preferably is 5 µg/mL.

16. The hydrogel according to any preceding claims wherein the positively charged immune molecule is surface-immobilized or in suspension.

17. A composition comprising the hydrogel according to any one of claims 1 to 16.

18. A hydrogel according to any one of claims 1 to 16 or a composition according to claim 17 for use as a medicament.

19. The hydrogel according to claim 18 for use in immunotherapy treatment, preferably in cancer treatment and autoimmune diseases.

20. Use of a hydrogel according to any one of claims 1 to 16 or a composition according to claim 17 in cell culture, preferably for T cell culture.

21. The use according to claim 20 wherein T cell is a CD4+ T cell.

22. A bioink comprising a hydrogel according to any one of claims 1 to 16 or a composition according to claim 17.

23. Use of the bioink according to claim 22 in 3D printing, preferably in 3D bioprinting.

24. A method for producing a hydrogel according to any one of claims 1 to 16 comprising the following steps:
(a) mixing a solution of functionalized PEG multi-arm star polymer with another solution of heparin, both solutions in buffer solution,
(b) incubating the solution obtained in step (a) at between 15°C to 45°C to gelify, and
(c) loading the hydrogel obtained in step (b) with at least one positively charged immune molecule.
